# EUROPÄISCHE PATENTSCHRIFT

(11) **EP 2 200 960 B1**
(45) Veröffentlichungstag und Bekanntmachung des Hinweises auf die Patenterteilung: **04.11.2015**
(21) Anmeldenummer: 08803385.7
(22) Anmeldetag: 29.08.2008
(51) Int. Cl.: C07C 29/60, C07C 31/20, B01J 23/72

(54) **VERFAHREN ZUR HERSTELLUNG VON 1,2-PROPANDIOL DURCH NIEDERDRUCK-HYDRIERUNG VON GLYCERIN**
METHOD FOR PRODUCING 1,2-PROPANDIOL BY LOW-PRESSURE HYDROGENATION OF GLYCERINE
PROCÉDÉ DE PRODUCTION DE PROPANE-1,2-DIOL PAR HYDROGÉNATION À BASSE PRESSION DE GLYCÉRINE

(30) Priorität: 31.08.2007 EP 07115463
(43) Veröffentlichungstag der Anmeldung: 30.06.2010
(73) Patentinhaber: BASF SE, 67056 Ludwigshafen (DE)
(72) Erfinder: HENKELMANN, Jochem, 68165 Mannheim (DE); PROCHAZKA, Roman, 68163 Mannheim (DE); BEY, Oliver, 67150 Niederkirchen (DE); MAURER, Stephan, 67435 Neustadt-Gimmeldingen (DE); STEINER, Jochen, 68165 Mannheim (DE); URTEL, Heiko, 68165 Mannheim (DE); THEIS, Gerhard, 67133 Maxdorf (DE); WAHL, Peter, 69118 Heidelberg (DE); BECKER, Michael, Singapore 228490 (SG)
(86) Internationale Anmeldenummer: PCT/EP2008/061387
(87) Internationale Veröffentlichungsnummer: WO 2009/027502

(56) Entgegenhaltungen:
- WO-A-2005/095536
- WO-A-2007/010299
- WO-A-2008/049470
- WO-A-2008/089899
- DE-A1- 4 302 464
- US-A- 5 616 817
- DATABASE WPI Section Ch, Week 200808 Thomson Scientific, London, GB; Class E17, AN 2008-B16826 XP002474619 CHEN C ET AL.: "Method of continuous preparation of 1, 2-propanediol with catalyst hydrogenation of glycerol" & CN 101 012 149 A (UNIV NANJING IND) 8. August 2007 (2007-08-08)

## Beschreibung

Die vorliegende Erfindung betrifft ein Verfahren zur Herstellung von 1,2-Propandiol, bei dem man einen glycerinhaltigen Strom, insbesondere einen großtechnisch bei der Herstellung von Biodiesel anfallenden Strom, einer Niederdruck-Hydrierung unterzieht.

Schwindende Erdölvorräte und steigende Kraftstoffpreise führen zu einem wachsenden Interesse, auf Erdölbasis hergestellte Kraftstoffe durch preiswerte und umweltschonende Alternativen zu ersetzen. Verfahren zur Herstellung von Kraftstoffen aus biogenen fett- oder ölhaltigen Ausgangsgemischen sowie beispielsweise in Restaurants anfallenden gebrauchten Ölen und tierischen Fetten sind seit längerem bekannt, wobei in Mitteleuropa derzeit überwiegend Rapsöl als Ausgangsprodukt bei der Produktion biogener Kraftstoffe eingesetzt wird. Biogene Öle und Fette selbst sind als Motorenkraftstoff weniger geeignet, da sie zuvor durch meist aufwändige Verfahren gereinigt werden müssen. Dazu zählt die Entfernung von Lecithinen, Kohlenhydraten und Proteinen, die Entfernung des so genannten Ölschlamms sowie die Entfernung der beispielsweise im Rapsöl in größeren Mengen enthaltenen freien Fettsäuren. So aufbereitete Pflanzenöle weichen dennoch von den technischen Eigenschaften herkömmlicher Dieselkraftstoffe in mehreren Punkten ab. So weisen sie in der Regel eine höhere Dichte als Dieselkraftstoff auf, die Cetanzahl von Rapsöl ist geringer als die von Dieselkraftstoff und die Viskosität ist gegenüber der von Dieselkraftstoff um ein Vielfaches höher. Dies führt zu einer nicht akzeptablen Verschlechterung der Kraftstoffeigenschaften, wie zu einem ungleichmäßigen Laufverhalten des Motors, einer deutlich erhöhten Geräuschemission sowie durch die höhere Viskosität zu einer schlechteren Zerstäubung und Verbrennung im Brennraum. Die Verwendung von reinen Pflanzenölen führt daher in herkömmlichen Motoren zu Verkokungen, verbunden mit erhöhter Partikelemission. Zur Lösung dieser Probleme ist es bekannt, die in den biogenen Öl- und Fettausgangsgemischen enthaltenen Triglyceride (Fettsäureester des Glycerins) in Fettsäuremonoalkylester, insbesondere Methyl- oder Ethylester, umzuwandeln. Diese auch als "Biodiesel" bezeichneten Ester lassen sich in der Regel ohne größere Nachrüstungen in Dieselmotoren einsetzen, wobei der Ausstoß von unverbrannten Kohlenwasserstoffen und Rußpartikeln im Vergleich zu normalem Dieselkraftstoff vielfach sogar reduziert werden kann. Bei der Umesterung der Triglyceride zur Biodieselherstellung fällt auch Glycerin an (≈ 10 %), welches sowohl aus Gründen der Wirtschaftlichkeit als auch der Nachhaltigkeit einer Verwertung zugeführt werden soll. Es besteht daher ein Bedarf an effektiven und wirtschaftlichen Verfahren, die auch eine Verwertung des bei der Biodieselherstellung anfallenden Glycerins ermöglichen. Diese Verfahren sollen sich insbesondere auch zur Verwertung weiterer großtechnisch zur Verfügung stehender Glycerinströme eignen.

Die US 2,360,844 beschreibt ein Verfahren zur Herstellung von Seifen, bei dem man ein rohes Glycerid mit C₁-C₄-Alkanolen umestert und dabei das freigesetzte Glycerin von den Monoalkylestern abtrennt. Die Verwertung des dabei erhaltenen Glycerins ist nicht beschrieben.

Die US 5,354,878 beschreibt ein Verfahren zur Herstellung von Niederalkylestern höherer Fettsäuren mit einem geringen Restglyceringehalt durch Umesterung von Fettsäuretriglyceriden und die Verwendung dieser Ester als Dieselkraftstoff.

Die DE 102 43 700 A1 beschreibt ein druckloses Verfahren zur Herstellung von Alkylestern höherer Fettsäuren, insbesondere Biodiesel, aus freie Fettsäuren enthaltenden Fettsäuretriglycerid-Ausgangsgemischen mit einer Kombination aus saurer Veresterung und basischer Umesterung. Das bei der Umesterung anfallende Glycerin wird zum Teil als Schleppmittel bei der Veresterung der freien Fettsäuren eingesetzt.

Es ist bekannt, höherwertige Alkohole durch katalytische Hydrierung in niedrigerwertige Alkohole zu überführen. So beschreibt die DE-PS-524 101 ein solches Verfahren, bei dem man u. a. Glycerin einer Gasphasenhydrierung in Gegenwart eines Hydrierungskatalysators mit Wasserstoff in erheblichem Überschuss unterzieht. Konkret werden zur Hydrierung von Glycerin mit Cr aktivierte Kupfer- oder Kobaltkatalysatoren eingesetzt.

Die DE-PS-541 362 beschreibt ein Verfahren zur Hydrierung von Polyoxyverbindungen, wie z. B. Glycerin, in Gegenwart von Katalysatoren bei erhöhten Temperaturen über 150 °C und unter erhöhtem Druck. Konkret wird die Hydrierung von Glycerin mit einem Nickelkatalysator bei einer Temperatur von 200 bis 240 °C und einem Wasserstoffdruck von 100 atm beschrieben.

R. Connor und H. Adkins beschreiben in J. Am. Chem. Soc. 54, 1932, S. 4678 - 4690 die Hydrogenolyse von sauerstoffhaltigen organischen Verbindungen, u. a. von 98%igem Glycerin, zu 1,2-Propandiol in Gegenwart eines Kupfer-Chrom-BariumOxid-Katalysators.

C. Montassier et al. beschreiben in Bulletin de la Societe Chimique de France 1989, Nr. 2, S. 148 - 155 Untersuchungen des Reaktionsmechanismus der katalytischen Hydrierung von Polyolen in Gegenwart diverser metallischer Katalysatoren, wie z. B. von Glycerin in Gegenwart von Raney-Kupfer.

J. Chaminand et al. beschreiben in Green Chem. 6, 2004, S. 359 - 361 die Hydrierung wässriger Glycerinlösungen bei 180 °C und 80 bar Wasserstoffdruck in Gegenwart von geträgerten Metallkatalysatoren auf Basis von Cu, Pd und Rh.

Die DE 43 02 464 A1 beschreibt ein Verfahren zur Herstellung von 1,2-Propandiol durch Hydrierung von Glycerin in Gegenwart eines heterogenen Katalysators bei Drücken von 20 bis 300 bar, insbesondere bei 100 bis 250 bar und Temperaturen von 150 °C bis 320 °C, wobei man Glycerin in dampfförmiger oder flüssiger Form über ein Katalysatorbett leitet. Als Katalysatoren werden u. a. Cu-Chromit, Cu-Zinkoxid, Cu-Aluminiumoxid und Cu-Siliciumdioxid genannt. Der Einsatz von glycerinhaltigen Strömen aus der Biodieselherstellung sowie Maßnahmen zur Vorbehandlung solcher Ströme vor ihrem Einsatz zur Hydrierung sind in diesem Dokument nicht beschrieben.

Die EP 0 523 015 beschreibt ein Verfahren zur katalytischen Hydrierung von Glycerin zur Herstellung von 1,2-Propandiol und 1,2-Ethandiol in Gegenwart eines Cu/Zn-Katalysators bei einer Temperatur von wenigstens 200 °C. Bei diesem Verfahren wird das Glycerin als wässrige Lösung mit einem Glyceringehalt von 20 bis 60 Gew.-% eingesetzt, der maximale Glyceringehalt in den Ausführungsbeispielen liegt bei 40 Gew.-%.

WO 2005/095536 beschreibt ein Niederdruckverfahren zur Umwandlung von Glycerin in Propylenglycol, bei dem man einen Glycerinhaltigen Strom mit einem Wassergehalt von höchstens 50 Gew.-% bei einer Temperatur im Bereich von 150 bis 250 °C und einem Druck im Bereich von 1 bis 25 bar einer katalytischen Hydrierung unterzieht.

M. A. Dasari et al. beschreiben in Appl. Catalysis A: General 281, 2005, S. 225 - 231 ein Verfahren zur Niederdruckhydrierung von Glycerin zu Propylenglycol bei einer Temperatur von 200 °C und einem Wasserstoffdruck von 200 psi (13,79 bar) in Gegenwart eines Nickel-, Palladium-, Platin-, Kupfer- oder Kupfer-Chromit-Katalysators. Es wurden unterschiedliche Reaktionsparameter getestet, wie u. a. der Wassergehalt des eingesetzten Glycerins. Dabei nahm mit abnehmendem Wassergehalt zwar der Umsatz zu, die höchste Selektivität wurde bei diesem Niederdruckverfahren jedoch bei einem Wassergehalt von 20 Gew.-% erzielt.

Die US 5,616,817 beschreibt ein Verfahren zur Herstellung von 1,2-Propandiol durch katalytische Hydrierung von Glycerin bei erhöhter Temperatur und erhöhtem Druck, bei dem man Glycerin mit einem Wassergehalt von höchstens 20 Gew.-% in Gegenwart eines Katalysators, der 40 bis 70 Gew.-% Kobalt, gegebenenfalls Mangan und/oder Molybdän und einen geringen Gehalt an Kupfer von 10 bis 20 Gew.-% enthält, umsetzt. Die Temperatur liegt dabei in einem Bereich von etwa 180 bis 270 °C und der Druck in einem Bereich von 100 bis 700 bar, vorzugsweise 200 bis 325 bar.

Die WO 2007/010299 A1 beschreibt ein Verfahren zur Hydrierung von Glycerin, bei dem man ein Glycerin-haltiges Ausgangsmaterial mit Wasserstoff in der Gasphase in Gegenwart eines Katalysators bei einer Temperatur von 160 bis 260°C, einem Druck von etwa 10 bis 30 bar, einem Verhältnis von Wasserstoff zu Glycerin von 400:1 bis 600:1 und einer Verweilzeit von etwa 0,01 bis 2,5 Stunden umsetzt.

Die CN 101012149 A beschreibt die kontinuierliche Herstellung von 1,2-Propandiol durch katalytische Hydrierung von Glycerin in Gegenwart eines Katalysators, der Kupfer, Zink, Mangan und/oder Aluminium enthält, bei einer Temperatur von 200-250°C und einem Druck von 25,5 bis 5 MPa.

Die WO 2005/095536 A2 beschreibt ein Verfahren zur Verwandlung von Glycerin zu Propylenglycol über eine Acetol-Zwischenstufe bei einer Temperatur von 150 bis 250°C und einem Druck von 1 bis 25 bar. Als Katalysator wird vorzugsweise ein Kupfer-Chromit-Pulver eingesetzt.

Die unveröffentlichte PCT/EP2007/051983 beschreibt ein Verfahren zur Herstellung von 1,2-Propandiol, bei dem man einen glycerinhaltigen Strom einer Hydrierung in Gegenwart eines kupferhaltigen, heterogenen Katalysators bei einer Temperatur von 100 bis 320 °C und einem Druck von 100 bis 325 bar unterzieht.

Es wurde nun überraschenderweise gefunden, dass auch glycerinhaltige Ströme mit hoher Glycerinkonzentration (d. h. einem niedrigen Wassergehalt) einer Niederdruck-Hydrierung zu 1,2-Propandiol mit hohen Ausbeuten und hoher Selektivität unterzogen werden können.

Gegenstand der Erfindung ist daher ein Verfahren zur Herstellung von 1,2-Propandiol, bei dem man
a) einen glycerinhaltigen Strom mit einem Wassergehalt von weniger als 20 Gew.-% bereitstellt, und
b) den glycerinhaltigen Strom einer Hydrierung in Gegenwart eines kupferhaltigen, heterogenen Katalysators bei einem Druck von weniger als 100 bar unterzieht
   wobei der Katalysator die folgenden Metalle in oxidischer Form, reduzierter Form (elementarer Form) oder einer Kombination davon umfasst:
   Cu, Al, wenigstens ein weiteres Metall, ausgewählt unter La, W, Mo, Ti, Zr.

Das in Schritt b) erhaltene Hydrierungsprodukt kann gegebenenfalls wenigstens einem Aufarbeitungsschritt unterzogen werden (Schritt c)).

Bevorzugt ist ein Verfahren, bei dem man den glycerinhaltigen Strom in Schritt b) einer Hydrierung bei einem Druck von 30 bis 99 bar, besonders bevorzugt bei einem Druck von 60 bis 96 bar, unterzieht.

Für den Einsatz in dem erfindungsgemäßen Verfahren eignen sich prinzipiell alle glycerinhaltigen Ströme, auch solche aus technisch ausgeübten Verfahren und mit den dabei anfallenden Reinheitsgraden. Dazu zählen insbesondere glycerinhaltige Ströme aus der Verarbeitung öl- und/oder fetthaltiger Ausgangsstoffe, z. B. aus der Seifenherstellung, der Fettsäure- und Fettsäureesterherstellung etc. Vorzugsweise handelt es sich bei dem in Schritt a) bereitgestellten glycerinhaltigen Strom um einen bei der Herstellung von Alkylestern höherer Fettsäuren durch Umesterung von Fettsäuretriglyceriden anfallenden glycerinhaltigen Strom, wie er insbesondere bei der Herstellung von "Biodiesel" anfällt. Diese Ausführungsform des erfindungsgemäßen Verfahrens wird im Folgenden noch näher beschrieben.

Der in Schritt a) eingesetzte glycerinhaltige Strom weist vorzugsweise einen Wassergehalt von höchstens 18 Gew.-%, bevorzugt von höchstens 15 Gew.-%, insbesondere von höchstens 10 Gew.-% auf. Besonders bevorzugt ist ein Wassergehalt entsprechend dem Glycerin-Monohydrat (Wassergehalt 16,3 Gew.-%) oder weniger. In einer speziellen Ausführungsform wird ein glycerinhaltiger Strom eingesetzt, der im Wesentlichen wasserfrei ist. Unter "im Wesentlichen wasserfrei" wird im Rahmen der vorliegenden Erfindung ein Wassergehalt von höchstens 3 Gew.-%, besonders bevorzugt von höchstens 1 Gew.-%, verstanden. Der Einsatz von glycerinhaltigen Strömen mit einem Wassergehalt im Bereich von weniger als 20 Gew.-% ermöglicht in dem zur Hydrierung eingesetzten Temperatur- und Druckbereich die Herstellung von 1,2-Propandiol mit hohen Ausbeuten und mit hoher Selektivität.

Die glycerinhaltigen Ströme können anstelle von oder zusätzlich zu Wasser wenigstens ein weiteres, vorzugsweise glycerinmischbares (und damit in der Regel auch wassermischbares), organisches Lösungsmittel aufweisen. Bevorzugt weisen die in Schritt a) bereitgestellten glycerinhaltigen Ströme einen Gesamtlösungsmittelgehalt von höchstens 20 Gew.-%, besonders bevorzugt höchstens 15 Gew.-%, insbesondere höchstens 10 Gew.-% und speziell höchstens 5 Gew.-% auf. Werden Lösungsmittelgemische eingesetzt, die Wasser und wenigstens ein glycerin- bzw. wassermischbares organisches Lösungsmittel enthalten, so beträgt der Anteil des organischen Lösungsmittels vorzugsweise höchstens 50 Gew.-%, besonders bevorzugt höchstens 20 Gew.-%, bezogen auf das Gesamtgewicht des Lösungsmittels. Geeignete glycerinmischbare organische Lösungsmittel sind C₁-C₄-Alkanole, wie Methanol, Ethanol, n-Propanol, Isopropanol, n-Butanol, tert.-Butanol, Polyole und Mono- und Dialkylether davon, cyclische Ether, wie Dioxan und Tetrahydrofuran, etc. Geeignete Lösungsmittel sind auch aromatische Kohlenwasserstoffe, wie Benzol, Toluol oder die Xylole. Bevorzugt als organische Lösungsmittel sind C₁-C₄-Alkanole, insbesondere Methanol und/oder Ethanol und deren Gemische mit Wasser. Bevorzugt weisen die in Schritt a) eingesetzten glycerinhaltigen Ströme jedoch keine organischen Lösungsmittel auf.

Die in Schritt a) bereitgestellten glycerinhaltigen Ströme können wenigstens einem Aufarbeitungsschritt unterzogen werden. Dazu zählt z. B. wenigstens ein Reinigungsschritt zur Entfernung unerwünschter Komponenten. Dazu zählt weiterhin eine Verringerung des Gehalts an Wasser und/oder, soweit vorhanden, organischer Lösungsmittel.

Je nach Herkunft können die glycerinhaltigen Ströme noch anorganische Salze als unerwünschte Komponente enthalten. Diese können aus dem Rohglycerin nach den im Folgenden beschriebenen Aufarbeitungsverfahren entfernt werden. Dazu eignet sich insbesondere eine thermische Aufarbeitung (z. B. unter Einsatz eines Sambay-Verdampfers).

Je nach Herkunft können die glycerinhaltigen Ströme auch Katalysatorgifte, d. h. Komponenten, welche die Hydrierung beeinträchtigen, indem sie den Hydrierungskatalysator deaktivieren, enthalten. Hierzu zählen z. B. stickstoffhaltige Verbindungen, wie Amine, und schwefelhaltige Verbindungen, wie Schwefelsäure, Schwefelwasserstoff, Thioalkohole, Thioether, z. B. Dimethylsulfid und Dimethyldisulfid, Kohlenoxidsulfid, Aminosäuren, z. B. Schwefel- und zusätzliche Stickstoffgruppen enthaltende Aminosäuren, Fettsäuren und deren Salz, etc. Zu den Katalysatorgiften zählen weiterhin Halogenverbindungen, Spuren von üblichen Extraktionsmitteln, z. B. Acetonitril oder N-Methylpyrrolidon, etc. sowie gegebenenfalls organische Phosphor- und Arsenverbindungen. Ein in glycerinhaltigen Strömen aus der Öl- und Fettveredelung häufig enthaltenes Katalysatorgift ist Schwefelsäure, welches als Katalysator bei der Veresterung bzw. Umesterung eingesetzt wird.

Zur Aufarbeitung der glycerinhaltigen Ströme in Schritt a) kann z. B. eine thermische Aufarbeitung, bevorzugt eine Destillation, eine Adsorption, ein Ionenaustausch, ein Membrantrennverfahren, eine Kristallisation, eine Extraktion oder eine Kombination aus zwei oder mehreren dieser Verfahren zum Einsatz kommen. Membrantrennverfahren unter Einsatz von Membranen definierter Porengrößen eignen sich speziell zur Verringerung des Wassergehalts und/oder zur Salzentfernung. Unter Kristallisation wird auch das partielle Ausfrieren der glycerinhaltigen Ströme an gekühlten Oberflächen verstanden. Somit lassen sich Verunreinigungen entfernen, die sich in der festen Phase anreichern.

In einer ersten Ausführung wird der glycerinhaltige Strom in Schritt a) einer Destillation zur Verringerung des Wassergehalts und/oder zur Entfernung von Komponenten, welche die katalytische Hydrierung beeinträchtigen, unterzogen. Diese kann prinzipiell nach üblichen, dem Fachmann bekannten Destillationsverfahren erfolgen. Geeignete Vorrichtungen zur destillativen Aufarbeitung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon. Die Entfernung von Schwefelsäure gelingt bereits durch eine einfache Destillation, insbesondere eine Kurzwegdestillation.

Geeignete Trennverfahren werden in den folgenden Dokumenten beschrieben: Sattler, Klaus: Thermische Trennverfahren, 3. Auflage, Wiley VCH, 2001; Schlünder E. U., Thurner F.: Destillation, Absorption, Extraktion, Springer Verlag, 1995; Mersmann, Alfons: Thermische Verfahrenstechnik, Springer Verlag, 1980; Grassmann P., Widmer F.: Einführung in die thermische Verfahrenstechnik, de Gruyter, 1997; Weiß S., Militzer K.-E., Gramlich K.: Thermische Verfahrenstechnik, Dt. Verlag für Grundstoffindustrie, Leipzig, Stuttgart, 1993. Auf diese Dokumente wird hier Bezug genommen.

In einer weiteren Ausführung wird der glycerinhaltige Strom in Schritt a) zur Verringerung des Gehalts an schwefelhaltigen Verbindungen, speziell schwefelhaltigen aromatischen Verbindungen, einer katalytischen Entschwefelung, gegebenenfalls in Gegenwart von Wasserstoff, unterzogen. Geeignete Entschwefelungsmittel umfassen eine Metallkomponente, wobei das Metall vorzugsweise ausgewählt ist unter Metallen der Gruppen 6, 7, 8, 9, 10, 11 und 12 des Periodensystems. Vorzugsweise werden die Metalle ausgewählt unter Mo, Ni, Cu, Ag, Zn und Kombinationen davon. Geeignete weitere Komponenten der Entschwefelungsmittel sind Dotierstoffe. Die Metallkomponente kann in oxidischer Form, reduzierter Form oder einer Mischung, die oxidierte und reduzierte Komponenten enthält, eingesetzt werden. Die aktive Komponente der Entschwefelungsmittel (Metallkomponente(n) und gegebenenfalls Dotierstoff(e)) können auf einen Träger aufgebracht werden. Geeignete Träger sind prinzipiell die im Folgenden genannten Adsorbentien und Katalysatorträger. Vorzugsweise ist das Trägermaterial ausgewählt unter Aktivkohle, Graphit, Ruß, Al₂O₃, SiO₂, TiO₂, ZrO₂, SiC, Silikaten, Zeolithen, Tonerden (z. B. Bentonite) und Kombinationen davon. Das Aufbringen wenigstens einer Metallkomponente und gegebenenfalls weiterer Komponenten auf ein Trägermaterial kann nach bekannten Verfahren erfolgen, z. B. durch (Co)-Präzipitation oder Imprägnieren. Die Entschwefelungsmittel können als Formkörper eingesetzt werden, z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern. Ungeträgerte Entschwefelungsmittel können nach üblichen Verfahren geformt werden, z. B. durch Extrudieren, Tablettieren, etc. Die Form geträgerter Entschwefelungsmittel wird durch die Form des Trägers bestimmt.

Zur katalytischen Entschwefelung wird vorzugsweise ein Entschwefelungsmittel eingesetzt, das Kupfer und Zink in einem Atomverhältnis von 1 : 0,3 bis 1 : 10, vorzugsweise 1 : 0,5 bis 1 : 3, insbesondere 1 : 0,7 bis 1 : 1,5, enthält. Bevorzugt wird ein Entschwefelungsmittel eingesetzt, das 35 bis 45 Gew.-% Kupferoxid, 35 bis 45 Gew.-% Zinkoxid und 10 bis 30 Gew.-% Aluminiumoxid enthält. In einer speziellen Ausführungsform handelt es sich bei dem Entschwefelungsmittel um eine zum Einsatz als Hydrierungskatalysator in Schritt b) befähigte Komponente. Diesbezüglich wird auf die folgende Offenbarung zu Hydrierkatalysatoren der zuvor genannten Zusammensetzung und Verfahren zu ihrer Herstellung Bezug genommen.

In einer Ausgestaltung dieser Verfahrensvariante werden die glycerinhaltigen Ströme in wenigstens einer Entschwefelungszone mit dem Entschwefelungsmittel in Kontakt gebracht und anschließend in wenigstens einer Reaktionszone hydriert.

Es versteht sich für den Fachmann, dass die konkrete Ausgestaltung und Anordnung der Entschwefelungs- und Reaktionszone(n) in jeder bekannten Weise erfolgen kann. Es ist möglich, die Entschwefelungs- und Reaktionszone(n) räumlich voneinander getrennt anzuordnen, d. h. durch die apparative Ausgestaltung baulich voneinander zu separieren oder auch in einer oder mehreren gemeinsamen Entschwefelungs-/Hydrierzone(n) zu verwirklichen.

Das Kupfer-Zink-Entschwefelungsmittel kann z. B. durch ein übliches Fällungs- oder Copräzipitationsverfahren erhalten und in oxidierter als auch in reduzierter Form eingesetzt werden.

In einer besonderen Ausführungsform enthält das Kupfer-Zink-Entschwefelungsmittel mindestens Kupfer, Zink und Aluminium, wobei das Kupfer: Zink : Aluminium-Atomverhältnis im Bereich von 1 : 0,3 : 0,05 bis 1 : 10 : 2, vorzugsweise bei 1 : 0,6 : 0,3 bis 1 : 3 : 1 und insbesondere bei 1 : 0,7 : 0,5 bis 1 : 1,5 : 0,9 liegt.

Zur Überführung in die reduzierte Form ist es möglich, das Entschwefelungsmittel einer Wasserstoffreduktion zu unterwerfen. Diese wird bei etwa 150 bis 350 °C, vorzugsweise bei etwa 150 bis 250 °C, in Gegenwart von Wasserstoff durchgeführt, wobei der Wasserstoff durch ein Inertgas, wie z. B. Stickstoff, Argon, Methan, insbesondere Stickstoff, verdünnt wird, so dass der Wasserstoffgehalt 10 Vol.-% oder weniger, vorzugsweise 6 Vol.-% oder weniger, insbesondere 0,5 bis 4 Vol.-%, beträgt. Das so erhaltene Kupfer-Zink-Entschwefelungsmittel ("reduzierte Form") kann in dieser Form in die Entschwefelung eingesetzt werden.

In einer Ausführungsform wird die Entschwefelung des glycerinhaltigen Stroms an dem Kupfer-Zink-Entschwefelungsmittel in oxidierter Form ohne Zusatz von Wasserstoff durchgeführt.

In einer weiteren Ausführungsform wird die Entschwefelung des glycerinhaltigen Stroms an dem Kupfer-Zink-Entschwefelungsmittel in oxidierter Form in Gegenwart von Wasserstoff durchgeführt.

In einer weiteren Ausführungsform wird die Entschwefelung des glycerinhaltigen Stroms an dem Kupfer-Zink-Entschwefelungsmittel in reduzierter Form ohne Zusatz von Wasserstoff durchgeführt.

In einer weiteren Ausführungsform wird die Entschwefelung des glycerinhaltigen Stroms an dem Kupfer-Zink-Entschwefelungsmittel in reduzierter Form in Gegenwart von Wasserstoff durchgeführt.

Üblicherweise wird die Entschwefelung in einem Temperaturbereich von 40 bis 200 °C, besonders bei 50 bis 180 °C, insbesondere bei 60 bis 160 °C, vorzugsweise bei 70 bis 120 °C, bei einem Druck von 1 bis 40 bar, besonders bei 1 bis 32 bar, vorzugsweise bei 1,5 bis 5 bar, insbesondere bei 2,0 bis 4,5 bar, durchgeführt. Die Entschwefelung kann in Gegenwart von Inertgasen, wie z. B. Stickstoff, Argon oder Methan, durchgeführt werden. In der Regel wird jedoch die Entschwefelung ohne Zugabe von Inertgasen durchgeführt.

Üblicherweise setzt man - soweit gewünscht - hierbei Wasserstoff mit einer Reinheit von ≥ 99,8 Vol.-%, insbesondere von ≥ 99,9 Vol.-%, vorzugsweise von ≥ 99,95 Vol.-%, ein. Diese Reinheitsgrade gelten analog für den Wasserstoff, der bei den gegebenenfalls durchgeführten Aktivierungen der Katalysatoren verwendet wird.

Üblicherweise liegt das Gewichtsverhältnis von glycerinhaltigem Strom zu Wasserstoff im Bereich von 40000 : 1 bis 1000 : 1, besonders im Bereich von 38000 : 1 bis 5000 : 1, insbesondere im Bereich von 37000 : 1 bis 15000 : 1, vorzugsweise im Bereich von 36000 : 1 bis 25000 : 1, im Speziellen im Bereich von 35000 : 1 bis 30000 : 1.

Der so entschwefelte glycerinhaltige Strom hat im Allgemeinen einen Gehalt von schwefelhaltigen Verunreinigungen, speziell von aromatischen Schwefelverbindungen, von höchstens 70 ppb, vorzugsweise von höchstens 50 ppb, und der Gesamtschwefelgehalt liegt bei insgesamt ≤ 200 ppb, vorzugsweise ≤ 150 ppb, insbesondere ≤ 100 ppb.

Die voranstehend beschriebenen Entschwefelungsmittel ermöglichen es auch, Chlor, Arsen und/oder Phosphor bzw. entsprechende chlor-, arsen- und/oder phosphorhaltige Verbindungen aus dem aromatischen Kohlenwasserstoff, oder dem Gemisch von aromatischen Kohlenwasserstoffen, zu reduzieren oder zu entfernen.

In einer weiteren Ausführung wird der glycerinhaltige Strom in Schritt a) zur Entfernung von Komponenten, welche die katalytische Hydrierung beeinträchtigen, mit wenigstens einem Adsorptionsmittel in Kontakt gebracht.

Die Adsorptionsmittel haben vorzugsweise eine spezifische Oberfläche, bestimmt nach BET, im Bereich von 10 bis 2000 m²/g, besonders bevorzugt im Bereich von 10 bis 1500 m²/g, insbesondere im Bereich von 10 bis 400 m²/g, speziell im Bereich von 60 bis 250 m²/g.

Geeignete Adsorptionsmittel sind z. B. aktive Aluminiumoxide. Ihre Herstellung erfolgt z. B. ausgehend von Aluminiumhydroxid, welches durch übliche Fällungsverfahren aus Aluminiumsalzlösungen erhältlich ist. Für das erfindungsgemäße Verfahren geeignete aktive Aluminiumoxide sind auch ausgehend von Aluminiumhydroxid-Gelen erhältlich. Zur Herstellung solcher Gele kann z. B. gefälltes Aluminiumhydroxid nach üblichen Aufarbeitungsschritten, wie Filtern, Waschen und Trocknen, aktiviert und anschließend gegebenenfalls vermahlen oder agglomeriert werden. Gewünschtenfalls kann man das resultierende Aluminiumoxid anschließend noch einem Formgebungsverfahren, wie Extrusion, Granulation, Tablettierung etc. unterwerfen. Als Adsorptionsmittel eignen sich vorzugsweise die Selexsorb TM-Typen der Firma Alcoa.

Geeignete Adsorptionsmittel sind weiterhin aluminiumoxidhaltige Feststoffe. Hierzu zählen z. B. die so genannten Tonerden, die als Hauptbestandteil ebenfalls Aluminiumoxide aufweisen.

Des Weiteren geeignete Adsorptionsmittel sind Aluminiumphosphate.

Weiterhin geeignete Adsorptionsmittel sind Siliciumdioxide, die z. B. durch Entwässerung und Aktivierung von Kieselgelen erhältlich sind. Ein weiteres Verfahren zur Herstellung von Siliciumdioxid ist die Flammhydrolyse von Siliciumtetrachlorid, wobei durch geeignete Variationen der Reaktionsparameter, wie z. B. der stöchiometrischen Zusammensetzung des Eduktgemisches und der Temperatur, die gewünschten Oberflächeneigenschaften des resultierenden Siliciumdioxids in weiten Bereichen variiert werden können.

Weiterhin geeignete Adsorptionsmittel sind Kieselgure, die ebenfalls als Hauptbestandteil Siliciumdioxide aufweisen. Dazu zählt z. B. die aus Kieselsedimenten gewonnene Diatomeenerde.

Weiterhin geeignete Adsorptionsmittel sind Titandioxide und Zirkoniumdioxide, wie sie z. B. in Römpp, Chemie-Lexikon, 9. Aufl. (Paperback), Bd. 6, S. 4629f. und S. 5156f. und der dort zitierten Literatur beschrieben sind. Hierauf wird hier in vollem Umfang Bezug genommen.

Weiterhin geeignete Adsorptionsmittel sind Phosphate, insbesondere kondensierte Phosphate, wie z. B. Schmelz- oder Glühphosphate, die eine große aktive Oberfläche aufweisen. Geeignete Phosphate sind z. B. in Römpp, Chemie-Lexikon, 9. Aufl. (Paperback), Bd. 4, S. 3376f. und der dort zitierten Literatur beschrieben. Hierauf wird hier in vollem Umfang Bezug genommen.

Weiterhin geeignete Adsorptionsmittel sind kohlenstoffhaltige Adsorbentien, bevorzugt Aktivkohle. Unter Aktivkohle versteht man hierbei im Allgemeinen Kohlenstoff mit poröser Struktur und hoher innerer Oberfläche. Zur Herstellung von Aktivkohle werden pflanzliche, tierische und/oder mineralische kohlenstoffhaltige Rohstoffe, z. B. mit Dehydratisierungsmitteln, wie Zinkchlorid oder Phosphorsäure, erhitzt oder durch trockene Destillation verkohlt und anschließend oxidativ aktiviert. Dazu kann man z. B. das verkohlte Material bei erhöhten Temperaturen von etwa 700 bis 1000 °C mit Wasserdampf, Kohlendioxid und/oder Gemischen davon behandeln.

Möglich ist auch ein Einsatz von Ionenaustauschern und/oder Adsorberharzen.

Die Adsorptionsmittel sind vorzugsweise unter Titandioxiden, Zirkoniumdioxiden, Siliciumdioxiden, Kieselgur, Aluminiumoxiden, aluminiumoxidhaltigen Feststoffen, Aluminiumphosphaten, natürlichen und synthetischen Aluminiumsilicaten, Phosphaten, kohlenstoffhaltigen Adsorbentien und Mischungen davon ausgewählt.

Die Adsorptionsmittel weisen im Allgemeinen eine spezifische Oberfläche, bestimmt nach BET, im Bereich von etwa 10 bis 2000 m²/g, insbesondere im Bereich von 10 bis 1500 m²/g und speziell im Bereich von 20 bis 600 m²/g, auf.

Zur adsorptiven Entfernung von unerwünschten Komponenten, insbesondere von Komponenten, welche die katalytische Hydrierung beeinträchtigen, wird der glycerinhaltige Strom in Schritt a) in einer Adsorptionszone mit wenigstens einem Adsorptionsmittel in Kontakt gebracht.

In einer speziellen Ausführungsform wird ein Adsorptionsmittel eingesetzt, welches wenigstens eine auch zum Einsatz als Hydrierungskatalysator in Schritt b) befähigte Komponente enthält. Auf die im Folgenden genauer beschriebenen Hydrierungskatalysatoren wird hier im vollen Umfang Bezug genommen. Zum Einsatz als Adsorptionsmittel eignen sich auch Kombinationen aus zwei oder mehr als zwei Adsorptionsmitteln. Dabei können sowohl ausschließlich auch als Hydrierungskatalysatoren befähigte Komponenten, ausschließlich nicht als Hydrierungskatalysatoren geeignete Adsorptionsmittel sowie Kombinationen davon eingesetzt werden.

In einer bevorzugten Ausführungsform setzt man als Adsorptionsmittel und als Hydrierungskatalysator die gleiche Komponente ein. Gegebenenfalls setzt man hierbei zusätzlich ein oder mehrere weitere, von dem Hydrierungskatalysator verschiedene herkömmliche Adsorptionsmittel, wie zuvor beschrieben, ein.

In einer Ausgestaltung des Verfahrens werden die glycerinhaltigen Ströme in wenigstens einer Adsorptionszone mit dem Adsorptionsmittel in Kontakt gebracht und anschließend in wenigstens einer Reaktionszone hydriert.

Es versteht sich für den Fachmann, dass die konkrete Ausgestaltung und Anordnung der Adsorptions- und Reaktionszone(n) in jeder bekannten Weise erfolgen kann. Es ist bevorzugt, die Adsorptions- und Reaktionszone(n) räumlich voneinander getrennt anzuordnen, d. h. durch die apparative Ausgestaltung baulich voneinander zu separieren.

Sofern man unterschiedliche Adsorptionsmittel verwendet, kann man z. B. eine erste Adsorptionszone in einem ersten Reaktor vorsehen, die ein erstes Adsorptionsmittel enthält, und separat, also apparativ getrennt davon, z. B. in einem zweiten Reaktor, eine zweite Adsorptionszone, die ein zweites Adsorptionsmittel enthält. Hierbei kann das erste und/oder das zweite Adsorptionsmittel wenigstens eine zum Einsatz als Hydrierungskatalysators befähigte Komponente enthalten.

In einer weiteren Ausführungsform setzt man ein herkömmliches Adsorptionsmittel zusammen mit einem zur Hydrierung befähigten Adsorptionsmittel in einer einzigen Adsorptionszone ein, z. B. in übereinander geschichteter Form, gemischt in Form einer statistischen Verteilung oder in Form einer Gradientenschüttung. Die Verwendung in gemischter Form erlaubt gegebenenfalls eine bessere Kontrolle der Temperatur. Im Fall einer Gradientenschüttung können lineare und nicht-lineare Gradienten verwendet werden. Es kann hierbei vorteilhaft sein, die Verteilung innerhalb der Schüttung derart vorzunehmen, dass der zu hydrierende glycerinhaltige Strom zunächst mit dem herkömmlichen Adsorptionsmittel in Kontakt gebracht wird, bevor er mit dem zur Hydrierung befähigten Adsorptionsmittel in Kontakt gebracht wird.

Vorteilhafterweise wird man wenigstens zwei Adsorptionszonen so anordnen, dass der zu hydrierende glycerinhaltige Strom in der ersten Adsorptionszone mit einem herkömmlichen Adsorptionsmittel in Kontakt gebracht wird und in der zweiten Adsorptionszone mit einem Adsorptionsmittel, das wenigstens eine zum Einsatz als Hydrierungskatalysator befähigte Komponente enthält, in Kontakt gebracht wird.

Die in Schritt a) des erfindungsgemäßen Verfahrens bereitgestellten glycerinhaltigen Ströme stammen bevorzugt aus der Herstellung von Biodiesel. Im Rahmen der vorliegenden Erfindung wird unter "Biodiesel" ein Gemisch aus Fettsäuremonoalkylestern verstanden, das aus biogenen öl- und/oder fetthaltigen Ausgangsgemischen gewonnen und in Dieselmotoren als Kraftstoff eingesetzt werden kann.

Zur Bereitstellung des glycerinhaltigen Stroms eigenen sich prinzipiell alle verfügbaren biogenen öl- und/oder fetthaltigen Ausgangsgemische. Öle und Fette sind allgemein feste, halbfeste oder flüssige Fettsäuretriglyceride, insbesondere aus pflanzlichen und tierischen Quellen, die chemisch im Wesentlichen aus Glycerinestern höherer Fettsäuren bestehen. Geeignete höhere Fettsäuren sind gesättigte oder ein- oder mehrfach ungesättigte Fettsäuren mit vorzugsweise 8 bis 40, besonders bevorzugt 12 bis 30 Kohlenstoffatomen. Dazu zählen z. B. n-Nonansäure, n-Decansäure, n-Undecansäure, n-Tridecansäure, Myristinsäure, Pentadecansäure, Palmitinsäure, Margarinsäure, Nonadecansäure, Arachinsäure, Behensäure, Lignocerinsäure, Cerotinsäure, Melissinsäure, Palmitoleinsäure, Oleinsäure, Linolsäure, Linolensäure, Stearinsäure, Elaostearinsäure, etc.

Pflanzliche Fette und Öle basieren im Wesentlichen auf Fettsäuren mit gerader Kohlenstoffatomanzahl, wohingegen tierische Fette und Öle auch Fettsäuren mit ungerader Kohlenstoffatomanzahl in freier oder als Triglyceridester gebundener Form enthalten können. Die in pflanzlichen Fetten und Ölen vorkommenden ungesättigten Fettsäuren liegen in der cis-Form vor, während tierische Fettsäuren häufig transkonfiguriert sind.

Zur Bereitstellung des glycerinhaltigen Stroms in Schritt a) können prinzipiell gebrauchte oder ungebrauchte, ungereinigte oder gereinigte pflanzliche, tierische oder technische Öle oder Fette oder Gemische davon eingesetzt werden. Diese können Anteile weiterer Inhaltsstoffe, z. B. freie Fettsäuren, enthalten. Der Anteil freier Fettsäuren beträgt im Allgemeinen 0 % bis 50 %, z. B. 0,1 bis 20 %, des zur Umesterung der Fettsäuretriglyceride eingesetzten Ausgangsgemischs. Freie Fettsäuren können gewünschtenfalls vor oder nach der Umesterung der Fettsäuretriglyceride entfernt werden. Salze dieser Fettsäuren (z. B. die Alkalisalze) können zuvor durch Ansäuern mit einer starken Säure, z. B. HCl, in die freie Säure überführt werden. Die Abtrennung der freien Fettsäuren gelingt z. B. durch Zentrifugieren. Vorzugsweise werden die in dem Ausgangsgemisch enthaltenen freien Fettsäuren ebenfalls in die Alkylester überführt. Dies kann vor, während oder nach der Umesterung der Fettsäuretriglyceride erfolgen.

Zur Bereitstellung des glycerinhaltigen Stroms in Schritt a) geeignete gebrauchte Fette und Öle sind fett- und/oder ölhaltige Komponenten, die nach ihrer Gewinnung aus entsprechenden biogenen Ausgangsmaterialien zunächst zu anderen Zwecken, z. B. zu technischen Zwecken oder Zwecken der Nahrungsmittelherstellung, verwendet wurden und die infolge dieser Verwendung chemisch modifiziert oder unmodifiziert sind oder zusätzliche Inhaltsstoffe, die insbesondere im Zusammenhang mit dieser Verwendung stehen, aufweisen können. Diese können gewünschtenfalls vor dem Einsatz zur Bereitstellung des glycerinhaltigen Stroms durch Umesterung zumindest teilweise entfernt werden. Zur Bereitstellung des glycerinhaltigen Stroms in Schritt a) geeignete ungebrauchte Fette und Öle sind fett- oder ölhaltige Komponenten, die nach ihrer Gewinnung aus den entsprechenden pflanzlichen oder tierischen Ausgangsmaterialien noch keinem anderen Zweck zugeführt wurden und die daher nur Inhaltsstoffe aufweisen, die aus den Ausgangsmaterialien stammen bzw. die mit der Gewinnung aus den Ausgangsmaterialien im Zusammenhang stehen. Auch aus diesen Ausgangsmaterialien können andere Inhaltsstoffe als Fettsäuretriglyceride (und gegebenenfalls freie Fettsäuren) gewünschtenfalls vor dem Einsatz zur Bereitstellung des glycerinhaltigen Stroms durch Umesterung zumindest teilweise entfernt werden.

Zur Aufreinigung und/oder Anreicherung können die ungebrauchten oder gebrauchten Fette oder Öle einer Entfernung von ungewünschten Inhaltsstoffen, wie Lecithinen, Kohlenhydraten, Proteinen, Ölschlamm, Wasser, etc. unterzogen werden.

Pflanzliche Öle und Fette sind solche, die zum überwiegenden Teil aus pflanzlichen Ausgangsmaterialien wie Samen, Wurzeln, Blättern oder anderen geeigneten Pflanzenteilen stammen. Tierische Fette oder Öle stammen zum überwiegenden Teil aus tierischen Ausgangsmaterialien, wie tierischen Organen, Geweben oder anderen Körperteilen oder Körperflüssigkeiten wie Milch. Technische Öle und Fette sind solche, die insbesondere aus tierischen oder pflanzlichen Ausgangsmaterialien gewonnen und für technische Zwecke aufbereitet wurden. Die erfindungsgemäß eingesetzten gebrauchten oder ungebrauchten, ungereinigten oder gereinigten Öle und/oder Fette sind insbesondere ausgewählt aus der Gruppe, bestehend aus Soapstock, Brown Grease, Yellow Grease, technischem Talg, technischem Schmalz, Frittierölen, Tierfett, Speisetalg, pflanzlichen Rohölen, tierischen Rohölen oder -fetten oder Gemischen davon.

Unter "Soapstock" wird ein bei der Verarbeitung von pflanzlichen Ölen anfallendes Nebenprodukt verstanden, insbesondere ein Nebenprodukt von Speiseöl-Raffinerien auf der Basis von Soja-, Rüb- oder Sonnenblumenöl. Soapstock weist einen Anteil von freien Fettsäuren von etwa 50 % bis 80 % auf.

Unter "Brown Grease" wird ein tierfetthaltiges Abfallprodukt verstanden, das einen Anteil von freien Fettsäuren von über 15 % bis 40 % aufweist. "Yellow Grease" enthält etwa 5 % bis 15 % freie Fettsäuren.

Unter "technischem Talg" und "technischem Schmalz" werden tierische Fette verstanden, die für technische Zwecke hergestellt und nach dem Trocken- oder Nassschmelzverfahren beispielsweise aus Schlachtabfällen gewonnen werden. Technische Talge werden nach ihrer Säurezahl bewertet und gehandelt, wobei der Gehalt an freien Fettsäuren je nach Herkunft z. B. zwischen 1 und 15 bis 20 Gew.-% und teilweise noch höher liegt.

Zu den "Tierfetten" gehören insbesondere bei der Verwertung von Geflügel-, Rinder-, Schweine-, Fisch- und Meeressäuger-Körpern abfallende fetthaltige Produkte, beispielsweise Solarstearin, ein fester Rückstand, der nach dem Auspressen von Schmalzöl aus Schweineschmalz verbleibt.

Die Bereitstellung des glycerinhaltigen Stroms in Schritt a) erfolgt vorzugsweise aus pflanzlichen Rohölen als Ausgangsmaterial. Dabei kann man von ungereinigten pflanzlichen Rohölen ausgehen, d. h. von flüssigen oder festen Zusammensetzungen, die aus pflanzlichen Ausgangsmaterialien z. B. durch Pressen gewonnen werden, wobei sie keine andere Behandlung erfahren haben als Absetzen in allgemein üblichen Zeiträumen und Abschleudern oder Filtrieren, bei dem zur Trennung des Öls von festen Bestandteilen nur mechanische Kräfte wie Schwerkraft, Fliehkraft oder Druck eingesetzt werden. Solche ungereinigten pflanzlichen Rohöle können auch durch Extraktion gewonnene pflanzliche Öle sein, wenn sich deren Eigenschaften von den entsprechenden, mittels Pressen gewonnenen pflanzlichen Ölen nicht oder nur unwesentlich unterscheiden. Der Anteil freier Fettsäuren in ungereinigten pflanzlichen Fetten und Ölen ist unterschiedlich und liegt z. B. bei etwa 0 bis 20 %, wie beispielsweise im Bereich von 0,1 bis 15 Gew.-% liegt. Der Gehalt an freien Fettsäuren kann jedoch auch mehr als 20 Gew.-% betragen.

Selbstverständlich können die pflanzlichen Öle vor ihrem Einsatz zur Umesterung einem oder mehreren Aufarbeitungsschritten unterzogen werden, wie sie im Folgenden noch genauer beschrieben sind. So können auch gereinigte pflanzliche Öle, beispielsweise Raffinate oder Halbraffinate, der vorstehend genannten pflanzlichen Öle als Ausgangsmaterialien eingesetzt werden.

Vorzugsweise wird zur Bereitstellung des glycerinhaltigen Stroms in Schritt a) ein pflanzliches Öl bzw. Fett eingesetzt, das vorzugsweise ausgewählt ist unter Rapsöl, Palmöl, Rüböl, Sojaöl, Sonnenblumenöl, Maiskeimöl, Baumwollsaatöl, Palmkern- und Kokosfett und Mischungen davon. Besonders bevorzugt wird Rapsöl oder ein rapsölhaltiges Gemisch eingesetzt.

Geeignet zur Bereitstellung des glycerinhaltigen Stroms in Schritt a) ist auch tierisches Öl bzw. Fett, das vorzugsweise ausgewählt ist unter Milchfett, Wollfett, Rindertalg, Schweineschmalz, Fischölen, Fischtran, etc. und Mischungen davon. Auch diese tierischen Fette oder Öle können vor ihrem Einsatz zur Umesterung einem oder mehreren Aufarbeitungsschritten unterzogen werden, wie sie im Folgenden noch genauer beschrieben sind.

Vorzugsweise umfasst die Bereitstellung des glycerinhaltigen Stroms in Schritt a) die folgenden Schritte:
a1) Bereitstellen eines biogenen fett- und/oder ölhaltigen Ausgangsgemisches,
a2) Umestern der in dem Ausgangsgemisch enthaltenen Fettsäuretriglyceride mit wenigstens einem C₁-C₉-Monoalkohol und gegebenenfalls Verestern der in dem Ausgangsgemisch enthaltenen freien Fettsäuren unter Bildung eines Veresterungsgemisches,
a3) Auftrennen des Veresterungsgemisches unter Erhalt wenigstens einer an Biodiesel angereicherten Fraktion und wenigstens einer an bei der Veresterung freigesetztem Glycerin angereicherten Fraktion,
a4) gegebenenfalls Aufreinigen der Glycerin angereicherten Fraktion.

### Schritt a1)

Die Bereitstellung des biogenen fett- und/oder ölhaltigen Ausgangsgemisches in Schritt a1) umfasst in einer bevorzugten Ausführungsform wenigstens einen Reinigungsschritt. Zur Reinigung kann das fett- und/oder ölhaltige Ausgangsgemisch wenigstens einem üblicherweise verwendeten Reinigungsverfahren für Fette und Öle, wie Klären, Filtration, Behandlung mit Bleicherden oder Behandlung mit Säuren oder Alkali zur Abtrennung störender Verunreinigungen wie Proteine, Phosphatide und Schleimstoffe sowie einer Kombination von wenigstens zwei dieser Reinigungsschritte unterzogen werden.

### Schritt a2)

Zur Umesterung der Fettsäuretriglyceride wird vorzugsweise wenigstens ein C₁-C₉-Monoalkohol, insbesondere wenigstens ein C₁-C₄-Monoalkohol eingesetzt. Bevorzugt ist der Einsatz von Methanol oder Ethanol.

Die Umesterung der Fettsäuretriglyceride kann sauer oder vorzugsweise basisch katalysiert erfolgen. Geeignete Säuren sind beispielsweise Mineralsäuren wie HCl, H₂SO₄ oder H₃PO₄.

Bevorzugt wird als Katalysator wenigstens eine Base eingesetzt. Diese ist vorzugsweise ausgewählt unter Alkalihydroxiden, wie NaOH und KOH, Erdalkalihydroxiden, wie Ca(OH)₂, Alkali- und Erdalkali-C₁-C₆-alkanolaten, wie NaOCH₃, KOCH₃, Na(OCH₂CH₂) und Ca(OCH₂CH₂)₂ und Mischungen davon. Besonders bevorzugt verwendet man NaOH, KOH oder NaOCH₃, ganz besonders bevorzugt NaOCH₃.

Die Menge der eingesetzten Base liegt üblicherweise im Bereich von 0,1 bis 10 Gew.-%, insbesondere von 0,2 bis 5 Gew.-%, bezogen auf die eingesetzte Menge an Fettsäuretriglyceriden.

Die Base wird bevorzugt in Form einer wässrigen oder alkoholischen, besonders bevorzugt alkoholischen, Lösung eingesetzt. Vorteilhafterweise verwendet man als Lösungsmittel für die Base das bereits für die Alkoholyse der Triglyceride eingesetzte Lösungsmittel. Bevorzugt wird zur Umesterung eine NaOCH₃-Lösung in Methanol eingesetzt.

Die Umesterung erfolgt vorzugsweise bei einer Temperatur von etwa 20 bis 150 °C, insbesondere 30 bis 95 °C.

Die Umesterung erfolgt in dafür üblichen, dem Fachmann bekannten Vorrichtungen. Nach einer geeigneten Ausführung erfolgt die Umesterung kontinuierlich. Vorzugsweise erfolgt die Umesterung in wenigstens einer Kolonne, wobei das erhaltene Umesterungsgemisch gleichzeitig einer Auftrennung unterzogen wird. Dabei wird im Allgemeinen eine höhersiedende Phase erhalten, die an dem basischen Katalysator, an nicht umgesetztem Monoalkohol und dem bei der Umesterung entstandenen Glycerin angereichert ist und eine niedrigersiedende Phase erhalten, die an dem Umesterungsprodukt angereichert ist. Sofern das Umesterungsprodukt noch nicht umgeesterte Triglyceride enthält, können diese ebenfalls abgetrennt und einer erneuten Umesterung in der ersten oder einer weiteren Umesterungsstufe unterzogen werden.

Anschließend wird das letzte Umesterungsgemisch in eine Trocknungsanlage überführt, wobei nochmals Restmengen an Wasser entfernt werden. Nach der Trocknung in der Trocknungsvorrichtung liegt das gewünschte Endprodukt Biodiesel in gereinigter Form vor und kann direkt als Kraftstoff eingesetzt werden.

Sofern das zur Bereitstellung des glycerinhaltigen Stroms in Schritt a) eingesetzte fett- und/oder ölhaltige Ausgangsgemisch freie Fettsäuren enthält, können diese vorzugsweise einer Veresterung zur Umwandlung in Biodiesel-taugliche Ester unterzogen werden.

Die freien Fettsäuren werden vorzugsweise mit dem gleichen C₁-C₉-Monoalkohol umgeestert, der zur Umesterung der Fettsäuretriglyceride eingesetzt wurde. Die Veresterung freier Fettsäuren kann vor, während oder nach der Umesterung der Fettsäuretriglyceride erfolgen. In einer bevorzugten Ausführung erfolgt die Veresterung freier Fettsäuren vor der Umesterung der Fettsäuretriglyceride.

Die Veresterung der freien Fettsäuren kann basisch oder vorzugsweise sauer katalysiert erfolgen. Geeignete Säuren sind die zuvor genannten Mineralsäuren, wie HCl, H₂SO₄ oder H₃PO₄, p-Toluolsulfonsäure, etc. Die Veresterung erfolgt vorzugsweise bei einer Temperatur von etwa 20 bis 95 °C, insbesondere 40 bis 80 °C.

Die Veresterung erfolgt in dafür üblichen, dem Fachmann bekannten Vorrichtungen. Dazu zählen Rührkessel und/oder Kolonnen, die gewünschtenfalls zu Kaskaden geschaltet sind. Bevorzugt erfolgt die Veresterung der freien Fettsäuren in wenigstens einer als Kolonne ausgeführten Veresterungseinrichtung, wobei das erhaltene Veresterungsgemisch gleichzeitig einer Auftrennung unterzogen wird. In einer geeigneten Ausführungsform erfolgt die Veresterung in Gegenwart eines Schleppmittels zur Erleichterung der Trennung.

### Schritt a3)

Während oder im Anschluss an die Umesterung und/oder Veresterung wird das Veresterungsgemisch einer Auftrennung unter Erhalt wenigstens einer an C₁-C₉-Monoalkylestern angereicherten Fraktion und wenigstens einer an bei der Umesterung freigesetztem Glycerin angereicherten Fraktion unterzogen. Die Auftrennung erfolgt vorzugsweise destillativ nach üblichen, dem Fachmann bekannten Verfahren. Geeignete Destillationsvorrichtungen sind die zuvor genannten.

### Schritt a4)

Die nach Auftrennen des Veresterungsgemisches in Schritt a3) erhaltene an Glycerin angereicherte Fraktion kann gegebenenfalls wenigstens einem Aufarbeitungsschritt unterzogen werden. Dazu zählt beispielsweise die Entfernung von unerwünschten Komponenten, wie Salzen, sowie von Komponenten, welche die katalytische Hydrierung beeinträchtigen oder die Abtrennung von Wasser und, falls vorhanden, organischem Lösungsmittel. Auf die vorherigen Ausführungen zu diesen Aufarbeitungsschritten wird in vollem Umfang Bezug genommen.

Bei den in dem erfindungsgemäßen Verfahren eingesetzten Katalysatoren kann es sich um Vollkatalysatoren oder geträgerte Katalysatoren handeln. Sie können in Form von einheitlich zusammengesetzten Katalysatoren, imprägnierten Katalysatoren, beschichteten Katalysatoren und Fällungskatalysatoren eingesetzt werden.

Eine weitere spezielle Ausführungsform von Katalysatoren, die sich besonders vorteilhaft für einen Einsatz in dem erfindungsgemäßen Verfahren eignen, sind Katalysatoren, die Kupfer in oxidischer Form sowie gegebenenfalls zusätzlich in elementarer Form enthalten. Der in Schritt b) eingesetzte Hydrierkatalysator enthält dann vorzugsweise wenigstens 23 Gew.-%, besonders bevorzugt wenigstens 35 Gew.-%, Kupfer in oxidischer und/oder elementarer Form, bezogen auf das Gesamtgewicht des Katalysators.

Ein häufig angewandtes Verfahren zur Herstellung solcher Katalysatoren besteht in der Tränkung von Trägermaterialien mit Lösungen der Katalysatorkomponenten, die anschließend durch thermische Behandlung, Zersetzung oder Reduktion in den katalytisch aktiven Zustand überführt werden.

Ein weiteres geeignetes Verfahren zur Herstellung von Katalysatoren umfasst die Fällung (Präzipitation) einer Katalysator-Komponente oder die Co-Fällung von zwei oder mehr als zwei Katalysator-Komponenten. So kann zur Herstellung eines Katalysatorformkörpers eine Kupferverbindung, gegebenenfalls wenigstens eine weitere Metallverbindung und/oder ein Additiv gefällt und anschließend einer Trocknung, Kalzination und Formung unterzogen werden. Die Fällung kann in Gegenwart eines Trägermaterials durchgeführt werden. Geeignete Ausgangsmaterialien für die Fällung sind Metallsalze und Metallkomplexe. Als Kupferverbindungen für die Fällung können prinzipiell alle bekannten Cu(I) und/oder Cu(II)-Salze eingesetzt werden, die in den zur Auftragung auf den Träger eingesetzten Lösungsmitteln löslich sind. Dazu zählen z. B. Nitrate, Carbonate, Acetate, Oxalate oder Ammoniumkomplexe. In einer bevorzugten Ausführung wird Kupfernitrat eingesetzt. Vorzugsweise wird zur Fällung ein wässriges Medium eingesetzt.

Geeignete wässrige Medien sind Substanzen oder Mischungen, die unter den Verfahrensbedingungen flüssig sind und die wenigstens 10 Gew.-%, besonders bevorzugt wenigstens 30 Gew.-%, insbesondere wenigstens 50 Gew.-%, Wasser enthalten. Der von Wasser verschiedene Anteil ist vorzugsweise ausgewählt unter anorganischen oder organischen Verbindungen, die wenigstens teilweise in Wasser löslich oder wenigstens teilweise mit Wasser mischbar sind. Beispielsweise sind die von Wasser verschiedenen Verbindungen ausgewählt unter organischen Lösungsmitteln, wie C₁-C₂₀-Alkanolen, insbesondere Methanol, Ethanol, n-Propanol, Isopropanol, n-Butanol, sec.-Butanol, tert.-Butanol, Penthanolen und Hexanolen, C₄-C₈-Cycloalkylethern, wie Tetrahydrofuranen, Pyranen, Dioxanen und Trioxanen, C₁-C₁₂-Dialkylethern, wie Dimethylether, Dibutylether und Methylbutylether. Das wässrige Medium enthält vorzugsweise weniger als 40 Gew.-%, besonders bevorzugt weniger als 30 Gew.-% und insbesondere weniger als 20 Gew.-% organische Lösungsmittel. In einer bevorzugten Ausführung des erfindungsgemäßen Verfahrens ist das wässrige Medium im Wesentlichen frei von organischen Lösungsmitteln.

Die Fällung kann durch bekannte Verfahren, z. B. Kühlen einer gesättigten Lösung, Zugabe eines Fällungsmittels, etc. induziert werden. Geeignete Fällungsmittel sind z. B. Säuren, Basen, Reduktionsmittel, etc.

Die Fällung kann durch Zugabe einer Säure oder einer Base zu dem wässrigen Medium induziert werden, das die Kupferverbindung und gegebenenfalls weitere Verbindungen enthält. Geeignete Säuren sind Mineralsäuren, wie HCl, H₂SO₄ und H₃PO₄. Die Base ist vorzugsweise ausgewählt unter Metalloxiden, Metallhydroxiden, insbesondere Alkalimetallhydroxiden, wie Natriumhydroxid und Kaliumhydroxid, Metallcarbonaten, insbesondere Alkalimetall- und Erdalkalimetallcarbonaten, wie Lithiumcarbonat, Natriumcarbonat, Kaliumcarbonat, Magnesiumcarbonat und Calciumcarbonat, Stickstoffbasen, insbesondere Ammoniak sowie primären, sekundären und tertiären Aminen.

Beispiele geeigneter Reduktionsmittel sind Carbonsäuren, wie Ameisensäure, Citronensäure, Milchsäure, Weinsäure und insbesondere Salze von Carbonsäuren, vorzugsweise die Alkalimetall-, Erdalkalimetall-, Ammonium- und C₁-C₁₀-Alkylammoniumsalze, phosphorige oder hypophosphorige Säure, die Salze von phosphoriger oder hypophosphoriger Säure, insbesondere die Alkalimetall- oder Erdalkalimetallsalze, C₁-C₁₀-Alkanole, wie Methanol, Ethanol und Isopropanol, Zucker, wie Aldosen und Ketosen in Form von Monosacchariden, Disacchariden und Oligosacchariden, insbesondere Glukose, Fruktose und Laktose, Aldehyde, wie Formaldehyd, Bor-Wasserstoff-Verbindungen, wie Borhydride, Borane, Metallboranate und Borankomplexe, wie Diboran, Natriumborhydrid und Aminoborane, insbesondere Trimethylaminoboran, Hydrazin und Alkylhydrazine, wie Methylhydrazin, Hydrogendithionite und Dithionite, insbesondere Natrium- und Kaliumhydrogendithionit, Natrium-, Kalium- und Zinkdithionite, Hydrogensulfide und Sulfide, insbesondere Natrium- und Kaliumhydrogensulfide, Natrium-Kalium- und Calciumsulfide, Hydroxylamin und Harnstoff, und Mischungen davon.

Die EP-A-0 434 062 beschreibt Hydrierungskatalysatoren, die durch Reduktion eines Precursors aus Oxiden des Kupfers, Aluminiums und wenigstens eines weiteren Metalls, ausgewählt unter Titan, Zirkon, erhältlich sind.

EP-A-552463 beschreibt in einer ersten Ausführungsform Hydrierkatalysatoren, wobei die oxidische Form im Wesentlichen der Zusammensetzung CuₐAl_{b}Zr_{c}Mn_{d}Oₓ entspricht, wobei die folgenden Beziehungen gelten: a > 0; b > 0; c ≥ 0; d > 0; a > b/2; b > a/4; a > c; a > d; und x die zur Wahrung der Elektroneutralität pro Formeleinheit erforderliche Anzahl von Sauerstoffionen bezeichnet. Nach einer weiteren Ausführungsform enthält der erfindungsgemäße Katalysator einen geringeren Anteil an Aluminiumoxid. Der Katalysator nach dieser Ausführungsform entspricht im Wesentlichen der Zusammensetzung CuₐAl_{b}Zr_{c}Mn_{d}Ox, wobei die folgenden Beziehungen gelten: a > 0; a/40 ≤ b ≤ a/4; c ≥ 0; d > 0; a > c; 0,5d ≤ a ≤ 0,95d und x die zur Wahrung der Elektroneutralität pro Formeleinheit erforderliche Anzahl von Sauerstoffionen bezeichnet.

Die WO 2006/005505 beschreibt Katalysatorformkörper, die sich für den Einsatz in dem erfindungsgemäßen Verfahren besonders eignen. Diese können durch ein Verfahren hergestellt werden, bei dem,
(i) ein oxidisches Material, umfassend Kupferoxid, Aluminiumoxid und mindestens eines der Oxide des Lanthans, Wolframs, Molybdäns, Titans oder Zirkoniums wobei die Oxide des Lanthans und/oder Wolframs bevorzugt sein sollen, bereitgestellt wird,
(ii) dem oxidischen Material pulverförmiges metallisches Kupfer, Kupferblättchen, pulverförmiger Zement oder deren Gemische oder ein Gemisch derselben mit Graphit zugegeben werden kann, und
(iii) das aus (ii) resultierende Gemisch zu einer Katalysatortablette oder einem Katalysatorextrudat mit einem Durchmesser d und/oder einer Höhe h < 6,0 mm, Katalysatorkugeln mit einem Durchmesser d < 6,0 mm oder Katalysator-Wabenkörper mit einem Zelldurchmesser r_{z} < 6,0 mm verformt wird.

Von den Oxiden des Lanthans, Wolframs, Molybdäns, Titans oder Zirkoniums ist Lanthanoxid bevorzugt. Die Zusammensetzung des oxidischen Materials ist im Allgemeinen so beschaffen, dass der Anteil an Kupferoxid im Bereich von 40 bis 90 Gew.-%, der Anteil an Oxiden des Lanthans, Wolframs, Molybdäns, Titans oder Zirkoniums im Bereich von 0 bis 50 Gew.-% und der Anteil an Aluminiumoxid im Bereich bis zu 50 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

In einer bevorzugten Ausführung umfasst das oxidische Material
(a) Kupferoxid mit einem Anteil im Bereich von 50 s x ≤ 80 Gew.-%, bevorzugt 55 ≤ x ≤ 75 Gew.%,
(b) Aluminumoxid mit einem Anteil im Bereich von 15 ≤ y ≤ 35 Gew.-%, bevorzugt 20 ≤ y ≤ 30 Gew.-%, und
(c) mindestens eines der Oxide des Lanthans, Wolframs, Molybdäns, Titans oder Zirkoniums, bevorzugt des Lanthans und/oder Wolframs, mit einem Anteil im Bereich von 2 ≤ z ≤ 20 Gew.-%, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, besonders 95 ≤ x + y + z ≤ 100.

Bevorzugte Katalysatoren umfassen die folgenden Metalle in oxidischer Form, reduzierter Form (elementarer Form) oder einer Kombination davon. Metalle, die in mehr als einer Oxidationsstufe stabil sind, können vollständig in einer der Oxidationsstufen oder in verschiedenen Oxidationsstufen eingesetzt werden:
Cu, Al, wenigstens ein weiteres Metall, ausgewählt unter La, W, Mo, Ti, Zr.

Besonders bevorzugte Katalysatoren umfassen die folgenden Metalle:
Cu, Al, La

Als inertes Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Aluminiumoxid und Siliciumdioxid. Als Siliciumdioxid-Trägermaterial können Siliciumdioxid-Materialien unterschiedlicher Herkunft und Herstellung, z. B. pyrogen erzeugte Kieselsäuren oder nasschemisch hergestellte Kieselsäuren, wie Kieselgele, Aerogele oder Fällungskieselsäuren, zur Katalysatorherstellung eingesetzt werden (zur Herstellung der verschiedenen SiO₂-Ausgangsmaterialien siehe: W. Büchner; R. Schliebs; G. Winter; K. H. Büchel: Industrielle Anorganische Chemie; 2. Aufl., S. 532 - 533, VCH Verlagsgesellschaft, Weinheim 1986).

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren, etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Die Katalysatorteilchen weisen im Allgemeinen einen Mittelwert des (größten) Durchmessers von 0,5 bis 20 mm, vorzugsweise 1 bis 10 mm, auf. Dazu zählen z. B. Katalysatoren in Form von Tabletten, z. B. mit einem Durchmesser von 1 bis 7 mm, vorzugsweise 2 bis 6 mm, und einer Höhe von 3 bis 5 mm, Ringen mit z. B. 4 bis 7 mm, vorzugsweise 5 bis 7 mm, Außendurchmesser, 2 bis 5 mm Höhe und 2 bis 3 mm Lochdurchmesser, oder Strängen unterschiedlicher Länge eines Durchmessers von z. B. 1,0 bis 5 mm. Derartige Formen können auf an sich bekannte Weise durch Tablettierung, Strangpressen oder Extrusion erhalten werden. Dazu können der Katalysatormasse übliche Hilfsmittel, z. B. Gleitmittel, wie Graphit, Polyethylenoxid, Cellulose oder Fettsäuren (wie Stearinsäure), und/oder Formhilfsmittel und Verstärkungsmittel, wie Fasern aus Glas, Asbest oder Siliciumcarbid, zugesetzt werden.

Eine spezielle Ausführungsform geträgerter Katalysatoren sind Schalenkatalysatoren. Bevorzugt eignen sich für das erfindungsgemäße Verfahren auch Schalenkatalysatoren. Schalenkatalysatoren umfassen eine schalenförmig auf einen Träger aufgebrachte katalytische Masse. Sie können in Form von Kugeln, Ringen, Zylindern, Würfeln, Quadern oder anderen geometrischen Körpern vorliegen. Unabhängig von der Art und Zusammensetzung des katalytisch aktiven Materials kann die Bereitstellung von Schalenkatalysatorteilchen prinzipiell erfolgen, indem man den Träger mit einem flüssigen Bindemittel und der katalytisch aktiven Masse in Kontakt bringt, dabei eine Schicht der Masse auf dem Träger aufbringt, und anschließend gegebenenfalls das Bindemittel teilweise entfernt. Dabei wird zur Bereitstellung der Katalysatorteilchen das katalytisch aktive Material bereits in seiner fertigen katalytisch aktiven Form, beispielsweise als calciniertes Mischoxid, aufgebracht. Geeignete Verfahren zur Herstellung von Schalenkatalysatoren sind z. B. in der DE-A-29 09 671 und in der EP-A-714 700 beschrieben. Nach dem letztgenannten Verfahren wird der Träger zunächst mit dem flüssigen Bindemittel befeuchtet, dann durch Inkontaktbringen mit trockener, feinteiliger, aktiver Katalysatormasse an der Oberfläche des befeuchteten Trägerkörpers eine Schicht aktiver Katalysatormasse angeheftet und anschließend gegebenenfalls das flüssige Bindemittel teilweise entfernt. In einer speziellen Ausführung werden die Schritte des Befeuchtens des Trägers, des Inkontaktbringens mit der Katalysatormasse und des Entfernens des flüssigen Bindemittels ein- oder mehrfach wiederholt, bis die gewünschte Schichtdicke des Schalenkatalysators erreicht ist.

Eine weitere spezielle Ausführungsform geträgerter Katalysatoren sind durch Tränkverfahren hergestellte Katalysatoren. Dazu können die katalytisch aktiven Katalysatorkomponenten oder Vorläuferverbindungen davon auf das Trägermaterial aufgetragen werden. Im Allgemeinen werden zum Tränken des Trägermaterials wässrige Salzlösungen der Komponenten, z. B. wässrige Lösungen von deren Halogeniden, Sulfaten, Nitraten, etc. aufgebracht. Die Kupferkomponente kann z. B. auch in Form einer wässrigen Lösung ihrer Aminkomplexsalze, beispielsweise als [Cu(NH₃)₄]SO₄- oder als [Cu(NH₃)₄](NO₃)₂-Lösung, gegebenenfalls in Gegenwart von Natriumcarbonat, auf das Trägermaterial aufgetragen werden. Selbstverständlich können auch andere Kupferaminkomplexe als die beispielhaft genannten mit gleichem Erfolg für die Katalysatorherstellung verwendet werden.

Die Imprägnierung des Trägermaterials mit den Vorläuferverbindungen der katalytisch aktiven Komponenten kann prinzipiell einstufig oder mehrstufig erfolgen. Die Imprägnierung kann in herkömmlichen Tränkvorrichtungen, z. B. Imprägniertrommeln, vorgenommen werden. Nach Trocknung und/oder Calcinierung erhält man dann den fertigen Katalysator. Die Trocknung der getränkten Katalysatorformkörper kann kontinuierlich oder chargenweise, z. B. in Band- oder Hordenöfen, erfolgen. Die Trocknung kann bei Atmosphärendruck oder vermindertem Druck erfolgen. Des Weiteren kann die Trocknung in einem Gasstrom, z. B. einem Luftstrom oder einen Stickstoffstrom, erfolgen. Je nach angewandtem Druck wird die Trocknung im Allgemeinen bei Temperaturen von 50 bis 200 °C, vorzugsweise 80 bis 150 °C durchgeführt. Die Calcinierung des gegebenenfalls zuvor getrockneten Katalysators erfolgt im Allgemeinen bei Temperaturen von 200 bis 800 °C, vorzugsweise 500 bis 700 °C. Die Calcinierung kann, wie die Trocknung, kontinuierlich oder chargenweise, z. B. in Band- oder Hordenöfen, durchgeführt werden. Die Calcinierung kann bei Atmosphärendruck oder vermindertem Druck und/oder in einem Gasstrom erfolgen, z. B. in einem Luft- oder einem Wasserstoffstrom. Eine Vorbehandlung mit Wasserstoff oder Wasserstoff enthaltenden Gasen im Allgemeinen unter Bedingungen, die den Hydrierbedingungen entsprechen, dient der Vorreduzierung/Aktivierung des Hydrierkatalysators. Der Katalysator kann aber auch in situ unter den bei der Hydrierung vorgegebenen Bedingungen, vorzugsweise unter Druck (z. B. bei einem Wasserstoffdruck von etwa 100 bis 325 bar), reduziert werden.

Bei der Hydrierung liegen das Glycerin und das resultierende 1,2-Propandiol vorzugsweise in flüssiger Phase vor.

Die Katalysatoren können beispielsweise in einem Festbett angeordnet sein oder als Suspension eingesetzt werden. Die Hydrierung kann dementsprechend z. B. in Rieselfahrweise oder in Sumpffahrweise durchgeführt werden. Zur Sumpfphasenhydrierung werden die Katalysatoren vorzugsweise in feinteiliger Form, z. B. als Pulver, in Suspension eingesetzt. Bei der Hydrierung der Rieselphase werden die Katalysatoren als Formkörper eingesetzt, wie sie zuvor beschrieben sind, z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern. Überschüssiger Wasserstoff wird vorzugsweise im Kreis geführt, wobei ein kleiner Teil als Abgas zur Entfernung von Inerten ausgeschleust werden kann. Es können dabei ein Reaktor oder mehrere Reaktoren eingesetzt werden, die in Reihe oder parallel zueinander geschaltet werden können.

Die Temperatur bei der Hydrierung in Schritt b) beträgt vorzugsweise 100 bis 320 °C, besonders bevorzugt 150 bis 300 °C, insbesondere 175 bis 250 °C.

Das Molverhältnis von Wasserstoff zu Glycerin beträgt vorzugsweise 1 : 1 bis 500 : 1, bevorzugt 1,1 : 1 bis 100 : 1.

Die Katalysator-Belastung bei kontinuierlicher Fahrweise beträgt vorzugsweise 0,1 bis 1, besonders bevorzugt 0,2 bis 0,6 kg, insbesondere 0,3 bis 0,6 kg zu hydrierendes Glycerin pro kg (Katalysator) pro h.

Der Umsatz, bezogen auf Glycerin, beträgt vorzugsweise wenigstens 80 %, insbesondere wenigstens 85 %. Die Selektivität bezogen auf 1,2-Propandiol beträgt bei dem erfindungsgemäßen Verfahren vorzugsweise wenigstens 85 %, besonders bevorzugt wenigstens 90 %. Vielfach können noch höhere Selektivitäten, von bis zu 95 % und darüber erzielt werden.

Die Hydrierung wird zweckmäßigerweise kontinuierlich durchgeführt. In einer speziellen Ausführung des erfindungsgemäßen Verfahrens wird der glycerinhaltige Strom in Schritt b) einer kontinuierlichen Hydrierung in wenigstens zwei (z. B. 2, 3 oder mehr als 3) hintereinander geschalteten Hydrierreaktoren unterzogen. Dies ermöglicht eine besonders vorteilhafte Abführung der entstehenden Hydrierwärme. Die ermöglicht weiterhin eine möglichst vollständige Hydrierung des Glycerins bei gleichzeitig guter Selektivität bezüglich des gewünschten 1,2-Propandiol. Zudem wird somit eine gegenüber aus dem Stand der Technik bekannten Verfahren geringere Katalysatormenge und/oder eine höhere Raum-Zeit-Ausbeute ermöglicht.

Erfolgt die Hydrierung kontinuierlich in wenigstens zwei hintereinander (in Reihe) geschalteten Hydrierreaktoren, so kann jeder Reaktor eine oder mehrere Reaktionszonen innerhalb des Reaktors aufweisen. Bei den Reaktoren kann es sich um gleiche oder verschiedene Reaktoren handeln. Diese können z. B. jeweils gleiche oder verschiedene Vermischungscharakteristiken aufweisen und/oder durch Einbauten ein- oder mehrfach unterteilt sein. Geeignete druckfeste Reaktoren für die Hydrierung sind dem Fachmann bekannt. Dazu zählen die allgemein üblichen Reaktoren für Gas-Flüssig-Reaktionen, wie z. B. Rohrreaktoren, Rohrbündelreaktoren, Gasumlaufreaktoren, Blasensäulen, Schlaufenapparate, Rührkessel (die auch als Rührkesselkaskaden ausgestaltet sein können), Air-Lift-Reaktoren etc.

Die verschiedenen Reaktoren können in Festbett- oder Suspensionsfahrweise betrieben werden. Die Festbettfahrweise ist dabei bevorzugt.

Erfolgt die Hydrierung in wenigstens zwei hintereinander geschalteten Hydrierreaktoren, so verfügt der erste Hydrierreaktor vorzugsweise über einen in einem externen Kreislauf geführten Strom (externer Umlaufstrom, Flüssigkeitsumlauf). Wird zur Hydrierung in Schritt b) eine Reaktorkaskade aus mehr als zwei (wie z. B. 3 oder mehr als 3) in Reihe geschalteten Reaktoren eingesetzt, so verfügen vorzugsweise alle in Reihe geschalteten Reaktoren mit Ausnahme des letzten über einen in einem externen Kreislauf geführten Strom. Vorzugsweise erfolgt die Umsetzung in dem letzten der Reihe geschalteten Reaktoren adiabatisch. Dieser Begriff wird im Rahmen der vorliegenden Erfindung im technischen und nicht im physiko-chemischen Sinne verstanden. So erfährt das Reaktionsgemisch beim Strömen durch den letzten Reaktor auf Grund der exothermen Hydrierungsreaktion in der Regel eine Temperaturerhöhung. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem zweiten Reaktor abgeführt, abgesehen von einem Restanteil, der durch natürliche Wärmeleitung und Wärmeabstrahlung vom Reaktor an die Umgebung abgegeben wird. Vorzugsweise wird der letzte Reaktor im geraden Durchgang betrieben.

In einer bevorzugten Ausführung werden zur Hydrierung in Schritt b) zwei hintereinander geschaltete Festbettreaktoren eingesetzt. Vorzugsweise verfügt der erste Hydrierreaktor dann über einen in einem externen Kreislauf geführten Strom. Des Weiteren wird die Umsetzung in dem zweiten Reaktor vorzugsweise adiabatisch durchgeführt. Der zweite Reaktor wird vorzugsweise im geraden Durchgang betrieben.

Der Hydrieraustrag besteht im Wesentlichen aus 1,2-Propandiol. Weitere Bestandteile sind u. a. Methanol, Ethanol, n-Propanol, Isopropanol, Propandiol-1,3, Glycerin, Ethylenglykol und Wasser. Der Hydrieraustrag kann anschließend nach üblichen, dem Fachmann bekannten Verfahren aufgearbeitet werden. Dazu eignen sich beispielsweise thermische Verfahren, vorzugsweise destillative Verfahren, Adsorption, Ionenaustausch, Membrantrennverfahren, Kristallisation, Extraktion oder eine Kombination von zwei oder mehreren dieser Verfahren. Bevorzugt wird der Hydrieraustrag durch Destillation aufgearbeitet. Dazu eignen sich übliche, dem Fachmann bekannte Destillationsverfahren. Geeignete Vorrichtungen für die destillative Aufarbeitung umfassen Destillationskolonnen, wie Bodenkolonnen, die mit Glocken, Siebplatten, Siebböden, Packungen, Einbauten, Ventilen, Seitenabzügen, etc. versehen sein können. Geeignet sind speziell Trennwandkolonnen, die mit Seitenabzügen, Rückführungen, etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, Fallfilmverdampfer, Sambay-Verdampfer, etc. und Kombinationen davon. Im Hydrieraustrag noch enthaltenes Glycerin kann, gegebenenfalls nach destillativer Abtrennung, in die Hydrierstufe zurückgeführt werden.

Die Erfindung wird anhand der folgenden, nicht einschränkenden Beispiele näher erläutert.

### Beispiele

Als Einsatzstoff für die Versuche zum Katalysatorscreening wurde Glycerin der Qualitäten Pharmaglycerin und Rein-Glycerin der Firma JCN Biodiesel Neckermann GmbH eingesetzt. Tabelle 1 zeigt die Analysedaten des eingesetzten Pharmaglycerins.

Die Analytik des Einsatzstoffes Glycerin sowie des Reaktionsaustrages erfolgt durch Gaschromatographie (Angaben in GC-Flächen-%).

| | |
|---|---|
| Gerät: | HP5890-2 mit Probensampler |
| Range: | 2 |
| Säule: | 30 m ZB5; Filmdicke 0,2 mm |
| Probenvolumen: | 0,2 µl |
| Trägergas: | Helium |
| Flussrate: | 100 ml/min |
| Injektortemperatur: | 290 °C |
| Detektor: | FID |
| Detektortemperatur: | 300 °C |
| Temperaturprogramm: | 150 - 280 °C mit 15 °C/min; Gesamtlaufzeit 10 min |

**Tabelle 1: Analytik des Pharmaglycerins**

| Wasser | Chlorid | Schwefel | Glyceringehalt | Verseifungszahl |
|---|---|---|---|---|
| [%] | [ppm] | [ppm] | [korr. FI.-%] | [mg KOH / g] |
| 0,09 | <1 | <0,1 | 99,8 | 1,4 |

Getestet wurden kupferhaltige Katalysatoren verschiedener Zusammensetzung (siehe Tabelle 2).

**Tabelle 2: Übersicht der getesteten Katalysatoren**

| Bezeichnung | Zusammensetzung |
|---|---|
| A | 67 % CuO; 5 % La₂O₃; Al₂O₃ (ad 100 %) + 15 % Cu |
| B ^{a)} | 43 % CuO; 18 % MgO: 0,8 % Cr₂O₃; 1 % ZnO; 35 % SiO₂ |
| C ^{a)} | 40 % CuO; 40 % ZnO; 20 % Al₂O₃ |
| D* ^{a)} | 40 % CuO; 40 % ZnO; 20 % Al₂O₃ |
| E ^{a)} | 61 % CuO; 39 % Al₂O₃ |
| F ^{a)} | 70 %CuO; 24,5 % ZnO; 5,5 % Al₂O₃ |
| G ^{a)} | 13 % CuO; 7 % CaO; 80 % SiO₂ |
| H ^{a)} | 55 % CuO/Al₂O₃ |
| I ^{a)} | 55 % CuO/Al₂O₃ (wie H jedoch reduziert) |
| K ^{a)} | 25 % CuO/SiO₂ |
| L ^{a)} | 16 % CuO/Al₂O₃/ZnO |
| M ^{a)} | 13 %CuO/28 % CoO/28 % NiO/31 % ZrO₂ |
| N ^{a)} | 70 % CuO/24,5 % ZnO/5,5 % Al₂O₃ |
| O ^{a)} | 100 % Cu (Raney-Cu)** |
| P ^{a)} | 40 % Cu/TiO₂ |

| | |
|---|---|
| * analog Katalysator C, jedoch höhere Calcinierungstemperatur ** von Fa. Aldrich ^{a)} nicht erfindungsgemäß | |

### Beispiele 1 und 2: kontinuierliche Hydrierung bei 60 bar Reaktordruck

Die zur Hydrierung eingesetzte Anlage besteht aus einem 100 ml Festbettreaktor, der mit einem Flüssigkeitsumlauf versehen ist, und der mit einem zweiten Festbettreaktor, der im geraden Durchlauf betrieben wird, in Reihe geschaltet ist. Bei Bedarf kann der Flüssigkeitsumlauf des ersten Reaktors zugeschaltet werden. Alle Anlagenteile sind aus Metall und für einen Betriebsdruck bis zu 250 bar ausgelegt. In den Reaktor wird waagengeregelt kontinuierlich die Glycerinlösung (wässrig 90%ig) eindosiert, welche bei definierten Bedingungen (Druck, Temperatur, Katalysator-Belastung) mit Wasserstoff zum Zielprodukt umgesetzt wird. Die Wasserstoff-Versorgung erfolgt aus 50 I-Stahlflaschen, die mit einem Druckluft-betriebenen Kompressor auf den benötigten Druck verdichtet werden. Der gewünschte Reaktionsdruck wird über eine Druckregelung im Abgasstrom eingestellt und die benötigte Menge Wasserstoff flussgeregelt über einen Massendurchflussmesser in den Reaktor eingespeist. Der flüssige Reaktoraustrag wird standgeregelt über eine HPLC-Pumpe ausgetragen und in einem Austragsbehälter gesammelt und analysiert. Bei allen Versuchen war der Katalysator stabil, und es trat kein Katalysatorverlust durch so genanntes "Leaching" auf.

**Tabelle 3: Reaktionsparameter:**

| Beispiel Nr. | 1 | 2 |
|---|---|---|
| Temperatur [°C] | 198 | 198 |
| Druck [bar] | 60 | 60 |
| Katalysator | A | A |
| Laufzeit [h] | 72 | 96 |
| K.B. [kg/l*h] | 0,24 | 0,16 |
| Zulauf [g/h] | 26,7 | 17,8 |
| RüFü [g/h] | 1300 | 1300 |

| | | |
|---|---|---|
| K.B. = Katalysatorbelastung RüFü = Flüssigkeitsrückführung (Umlauf) | | |

**Tabelle 4: GC-Analytik**

| Beispiel Produkte [FI.-%] | 1 | 2 |
|---|---|---|
| Methanol | 0,26 | 0,3 |
| 2-Propanol | 0,13 | 0,18 |
| 1-Propanol | 1,28 | 1,71 |
| Ethylenglykol | 1,95 | 2,25 |
| 1,2-Propandiol | 83,68 | 86,4 |
| Glycerin | 12,52 | 8,87 |
| Umsatz | 87,48 | 91,13 |

### Beispiele 3 bis 7:

### (Vergleichsbeispiele mit Glycerin mit einem Wassergehalt von 50 Gew.-%)

Zur Umsetzung wird eine Vorrichtung aus 16 unabhängig betriebenen "Slurry-Phase" Reaktoren der Fa. AMTEC eingesetzt, die eine parallele Durchführung mehrerer Hydrierversuche ermöglicht. Jeder Reaktor weist ein Volumen von 15 mL auf, ist magnetisch gerührt und ist für eine maximale Temperatur Tₘₐₓ von 250 °C und einen maximalen Druck Pₘₐₓ von 95 bar ausgelegt. Es wird jeweils eine Aufschlämmung von 0,1 g Katalysator P (40 % Cu/TiO₂) eingesetzt. Jeder Reaktor wird 5-mal mit 20 bar Stickstoff beschickt und anschließend auf 5 bar entspannt. Der Katalysator wird danach 10 Stunden mit Wasserstoff vorbehandelt und anschließend 5 mL einer 50%igen wässrigen Glycerinlösung zugegeben und 10 Stunden hydriert. Dazu wird unter Rühren die Temperatur bis zum Erreichen der Endtemperatur erhöht und Wasserstoff bis zum Erreichen des Enddrucks von 95 bar zugegeben. Druck und Temperatur werden während der Hydrierung konstant gehalten. Nach Abschluss der Reaktion werden die Reaktoren entspannt und auf Umgebungstemperatur abkühlen gelassen. Der Reaktoraustrag wird mittels GC, wie zuvor beschrieben, analysiert.

**Tabelle 5:**

| Bsp. Nr. | Temperatur [°C] | GC-Flächen-% | | | | | Umwandlung % | 1,2-PDO Selektivität % |
|---|---|---|---|---|---|---|---|---|
| | | EtOH | PrOH | Hydroxyac. | 1,2-PDO | Glyc. | | |
| 3 | 220 | 0,74 | 0,74 | 0,18 | 25,67 | 70,02 | 29,98 | 85,62 |
| 4 | 205 | 0,02 | 0,01 | 0,05 | 7,29 | 90,40 | 9,60 | 76,01 |
| 5 | 190 | 0,01 | 0,00 | 0,00 | 1,82 | 94,66 | 5,34 | 34,16 |
| 6 | 175 | 0,01 | 0,00 | 0,00 | 0,76 | 96,19 | 3,81 | 19,84 |
| 7 | 160 | 0,01 | 0,00 | 0,00 | 0,21 | 97,29 | 2,71 | 7,62 |

| | | | | | | | | |
|---|---|---|---|---|---|---|---|---|
| EtOH Ethanol PrOH Propanol Hydroxyac. Hydroxyaceton 1,2-PDO 1,2-Propandiol Glyc. Glycerin | | | | | | | | |

### Beispiele 8 bis 19:

### (Vergleichsbeispiele mit Glycerin mit einem Wassergehalt von 50 Gew.-%)

Unter den für die Beispiele 3 bis 7 angegebenen Bedingungen wurden die in der Tabelle 6 zusammengefassten Hydrierversuche jeweils mit 0,1 g verschiedener Katalysatoren durchgeführt.

**Tabelle 6:**

| Bsp. Nr. | Katalysator | Katalysatormenge [g] | T[°C] | GC-Flächen-% | | | | | Umwandlung % | 1,2-PDO Selektivität % |
|---|---|---|---|---|---|---|---|---|---|---|
| | | | | EtOH | PrOH | Hydroxyac. | 1,2-PDO | Glyc. | | |
| 8 | P | 0,1 | 220 | 0,88 | 0,77 | 0,03 | 30,83 | 59,75 | 40,25 | 76,61 |
| 9 | E | 0,1 | 220 | 1,23 | 0,59 | 0,23 | 9,48 | 85,31 | 14,69 | 64,50 |
| 10 | M | 0,1 | 220 | 0,37 | 1,07 | 0,02 | 31,17 | 56,17 | 43,83 | 71,10 |
| 11 | H | 0,1 | 220 | 0,06 | 0,03 | 0,13 | 53,38 | 45,55 | 54,45 | 98,04 |
| 12 | N | 0,1 | 220 | 0,09 | 0,50 | 0,47 | 23,65 | 67,94 | 32,06 | 73,77 |
| 13 | E | 0,1 | 190 | 0,01 | 0,00 | 0,18 | 2,77 | 93,53 | 6,47 | 42,81 |
| 14 | M | 0,1 | 190 | 1,94 | 0,00 | 0,01 | 3,87 | 87,72 | 12,28 | 31,52 |
| 15 | H | 0,1 | 190 | 0,01 | 0,00 | 0,14 | 9,37 | 88,76 | 11,24 | 83,35 |
| 16 | P | 0,1 | 160 | 0,04 | 0,00 | 0,12 | 1,95 | 95,78 | 4,22 | 46,22 |
| 17 | E | 0,1 | 160 | 0,00 | 0,00 | 0,12 | 1,56 | 96,33 | 3,67 | 42,43 |
| 18 | M | 0,1 | 160 | 7,08 | 0,00 | 0,00 | 1,42 | 88,12 | 11,88 | 11,92 |
| 19 | H | 0,1 | 160 | 0,00 | 0,00 | 0,15 | 2,92 | 94,15 | 5,85 | 49,94 |

| | | | | | | | | | | |
|---|---|---|---|---|---|---|---|---|---|---|
| T Temperatur EtOH Ethanol PrOH Propanol Hydroxyac. Hydroxyaceton 1,2-PDO 1,2-Propandiol Glyc. Glycerin | | | | | | | | | | |

### Beispiele 20 bis 22: (nicht erfindungsgemäß)

### (Cu-haltiger Katalysator im kontinuierlichen Festbettreaktor mit Rückführung)

Es wird eine Aufschlämmung von 70 mL Katalysator P (40 % Cu/TiO₂) in den Reaktor eingesetzt. Der Reaktor wird mit Stickstoff gespült und danach der Katalysator mit Wasserstoff behandelt. Dann gibt man kontinuierlich eine 90%ige wässrige Glycerinlösung in einer Menge von 31,11 g/h in den Reaktor. Die gewünschte Rückführung wird eingestellt (siehe Tabelle 7). Die erhaltene Katalysatorbeschickung beträgt 0,4 kg Glycerin/L x h⁻¹. Der Reaktoraustrag wird mittels GC, wie zuvor beschrieben, analysiert.

**Tabelle 7:**

| Bsp Nr. | Dauer [h] | T [°C] | Druck p [bar] | Rückführung [g/h] | GC-Flächen% | | | | | | Umwandlung % | 1,2'-PDO Selektivität % |
|---|---|---|---|---|---|---|---|---|---|---|---|---|
| | | | | | EtOH | PrOH | Hydroxyac. | 1,2-PDO | EG | Glyc. | | |
| 20 | 127 | 217 | 40 | 155 | 0,13 | 0,35 | 0,66 | 81,14 | 0,00 | 15,30 | 84,70 | 95,80 |
| 21 | 141 | 217 | 40 | 155 | 0,51 | 0,70 | 0,68 | 76,14 | 0,00 | 18,82 | 81,18 | 93,80 |
| 22 | 213 | 217 | 100 | 156 | 0,66 | 0,43 | 0,30 | 83,40 | 0,06 | 13,94 | 86,06 | 96,91 |

| | | | | | | | | | | | | |
|---|---|---|---|---|---|---|---|---|---|---|---|---|
| T Temperatur Hydroxyac. Hydroxyaceton EtOH Ethanol 1,2-PDO 1,2-Propandiol PrOH Propanol Glyc. Glycerin EG Ethylenglycol | | | | | | | | | | | | |

## Patentansprüche

1. Verfahren zur Herstellung von 1,2-Propandiol, bei dem man
a) einen glycerinhaltigen Strom mit einem Wassergehalt von weniger als 20 Gew.-% bereitstellt, und
b) den glycerinhaltigen Strom einer Hydrierung in Gegenwart eines kupferhaltigen, heterogenen Katalysators bei einem Druck von weniger als 100 bar unterzieht, wobei der Katalysator die folgenden Metalle in oxidischer Form, reduzierter Form oder einer Kombination davon umfasst: Cu, Al, wenigstens ein weiteres Metall, ausgewählt unter La, W, Mo, Ti, Zr.

2. Verfahren nach Anspruch 1, bei dem man den glycerinhaltigen Strom in Schritt b) einer Hydrierung bei einem Druck im Bereich von 30 bis 99 bar, vorzugsweise bei einem Druck im Bereich von 60 bis 96 bar, unterzieht.

3. Verfahren nach einem der vorhergehenden Ansprüche, bei dem man in Schritt a) einen bei der Herstellung von Alkylestern höherer Fettsäuren durch Umesterung von Fettsäuretriglyceriden anfallenden glycerinhaltigen Strom bereitstellt.

4. Verfahren nach einem der Ansprüche 1 bis 3, wobei der glycerinhaltige Strom einen Wassergehalt von höchstens 18 Gew.-%, bevorzugt von höchstens 15 Gew.-%, insbesondere von höchstens 10 Gew.-%, aufweist.

5. Verfahren nach einem der Ansprüche 1 bis 3, wobei der glycerinhaltige Strom einen Wassergehalt von höchstens 3 Gew.-% aufweist.

6. Verfahren nach einem der vorhergehenden Ansprüche, bei dem man in Schritt a) den glycerinhaltigen Strom einer Aufarbeitung durch wenigstens ein Aufarbeitungsverfahren unterzieht, das ausgewählt ist unter thermischer Aufarbeitung, Adsorption, Ionenaustausch, Membrantrennung, Kristallisation, Extraktion oder eine Kombination aus zwei oder mehreren dieser Verfahren.

7. Verfahren nach einem der vorhergehenden Ansprüche, bei dem man in Schritt a) den glycerinhaltigen Strom einer Destillation zur Verringerung des Wassergehalts Geänderte Seite und/oder zur Entfernung von Komponenten, welche die katalytische Hydrierung beeinträchtigen, unterzieht.

8. Verfahren nach einem der vorhergehenden Ansprüche, bei dem man in Schritt a) den glycerinhaltigen Strom zur Verringerung des Gehalts an schwefelhaltigen Verbindungen, speziell schwefelhaltigen aromatischen Verbindungen, einer katalytischen Entschwefelung, gegebenenfalls in Gegenwart von Wasserstoff, unterzieht.

9. Verfahren nach einem der vorhergehenden Ansprüche, bei dem man in Schritt a) den glycerinhaltigen Strom zur Entfernung von Komponenten, welche die katalytische Hydrierung beeinträchtigen, mit wenigstens einem Adsorptionsmittel in Kontakt bringt.

10. Verfahren nach einem der vorhergehenden Ansprüche, wobei die Bereitstellung des glycerinhaltigen Stroms in Schritt a) die folgenden Schritte umfasst:
a1) Bereitstellen eines biogenen fett- und/oder ölhaltigen Ausgangsgemisches,
a2) Umestern der in dem Ausgangsgemisch enthaltenen Fettsäuretriglyceride mit wenigstens einem C₁-C₉-Monoalkohol und gegebenenfalls Verestern der in dem Ausgangsgemisch enthaltenen freien Fettsäuren unter Bildung eines Veresterungsgemisches,
a3) Auftrennen des Veresterungsgemisches unter Erhalt wenigstens einer an C₁-C₉-Monoalkylestern angereicherten Fraktion und wenigstens einer an bei der Umesterung freigesetztem Glycerin angereicherten Fraktion,
a4) gegebenenfalls Aufreinigen der an Glycerin angereicherten Fraktion.

11. Verfahren nach einem der Ansprüche 1 bis 10, wobei der in Schritt b) eingesetzte Katalysator die folgenden Metalle umfasst: Cu, Al, La.

12. Verfahren nach einem der vorhergehenden Ansprüche 1 bis 10, wobei der in Schritt b) eingesetzte Hydrierkatalysator wenigstens 23 Gew.-%, bevorzugt wenigstens 35 Gew.-%, Kupfer, in oxidischer und/oder elementarer Form, bezogen auf das Gesamtgewicht des Katalysators, enthält.

13. Verfahren nach einem der vorhergehenden Ansprüche, bei dem man den glycerinhaltigen Strom in Schritt b) einer kontinuierlichen Hydrierung in wenigstens zwei hintereinander geschalteten Hydrierreaktoren unterzieht.

14. Verfahren nach Anspruch 13, wobei zur Hydrierung in Schritt b) zwei hintereinander geschaltete Festbettreaktoren eingesetzt werden.

15. Verfahren nach Anspruch 14, wobei der erste Hydrierreaktor über einen in einem externen Kreislauf geführten Strom aus der Reaktionszone verfügt.

16. Verfahren nach einem der Ansprüche 14 oder 15, wobei die Umsetzung in dem zweiten Reaktor adiabatisch durchgeführt wird.

## Claims

1. A process for preparing 1,2-propanediol, in which
a) a glycerol-containing stream having a water content of less than 20% by weight is provided, and
b) the glycerol-containing stream is subjected to a hydrogenation in the presence of a heterogeneous copper catalyst at a pressure of less than 100 bar, the catalyst comprising the following metals in oxidic form, reduced form or a combination thereof: Cu, Al, at least one further metal selected from La, W, Mo, Ti, Zr.

2. The process according to claim 1, wherein the glycerol-containing stream is subjected in step b) to a hydrogenation at a pressure in the range from 30 to 99 bar, preferably at a pressure in the range from 60 to 96 bar.

3. The process according to either of the preceding claims, in which a glycerol-containing stream which is obtained in the preparation of alkyl esters of higher fatty acids by transesterification of fatty acid triglycerides is provided in step a).

4. The process according to any of claims 1 to 3, wherein the glycerol-containing stream has a water content of at most 18% by weight, preferably of at most 15% by weight, especially of at most 10% by weight.

5. The process according to any of claims 1 to 3, wherein the glycerol-containing stream has a water content of at most 3% by weight.

6. The process according to any of the preceding claims, in which the glycerol-containing stream is subjected in step a) to a workup by at least one workup process which is selected from thermal workup, adsorption, ion exchange, membrane separation, crystallization, extraction or a combination of two or more of these processes.

7. The process according to any of the preceding claims, in which the glycerol-containing stream is subjected in step a) to a distillation to reduce the water content and/or to remove components which impair the catalytic hydrogenation.

8. The process according to any of the preceding claims, in which the glycerol-containing stream is subjected in step a) to a catalytic desulfurization, optionally in the presence of hydrogen, to reduce the content of sulfur compounds, especially aromatic sulfur compounds.

9. The process according to any of the preceding claims, in which the glycerol-containing stream is contacted in step a) with at least one adsorbent to remove components which impair the catalytic hydrogenation.

10. The process according to any of the preceding claims, wherein the provision of the glycerol-containing stream in step a) comprises the following steps:
a1) providing a biogenic fat- and/or oil-containing starting mixture,
a2) transesterifying the fatty acid triglycerides present in the starting mixture with at least one C₁-C₉ monoalcohol and optionally esterifying the free fatty acids present in the starting mixture to form an esterification mixture,
a3) separating the esterification mixture to obtain at least one fraction enriched in C₁-C₉ monoalkyl esters and at least one fraction enriched in glycerol released in the transesterification,
a4) optionally purifying the glycerol-enriched fraction.

11. The process according to any of claims 1 to 10, wherein the catalyst used in step b) comprises the following metals: Cu, Al, La.

12. The process according to any of the preceding claims 1 to 10, wherein the hydrogenation catalyst used in step b) comprises at least 23% by weight, preferably at least 35% by weight, of copper, in oxidic and/or elemental form, based on the total weight of the catalyst.

13. The process according to any of the preceding claims, in which the glycerol-containing stream is subjected in step b) to a continuous hydrogenation in at least two hydrogenation reactors connected in series.

14. The process according to claim 13, wherein two fixed bed reactors connected in series are used for the hydrogenation in step b).

15. The process according to claim 14, wherein the first hydrogenation reactor possesses a stream out of the reaction zone conducted in an external circuit.

16. The process according to either of claims 14 and 15, wherein the reaction in the second reactor is performed adiabatically.

## Revendications

1. Procédé pour la préparation de 1,2-propanediol, dans lequel
a) on met à disposition un flux contenant du glycérol présentant une teneur en eau inférieure à 20% en poids et
b) on soumet le flux contenant du glycérol à une hydrogénation en présence d'un catalyseur hétérogène, contenant du cuivre à une pression inférieure à 100 bars, le catalyseur comprenant les métaux suivants sous forme oxyde, sous forme réduite ou sous forme d'une combinaison correspondante :
Cu, Al, au moins un autre métal, choisi parmi La, W, Mo, Ti, Zr.

2. Procédé selon la revendication 1, dans lequel on soumet le flux contenant du glycérol dans l'étape b) à une hydrogénation à une pression dans la plage de 30 à 99 bars, de préférence à une pression dans la plage de 60 à 96 bars.

3. Procédé selon l'une quelconque des revendications précédentes, dans lequel, dans l'étape a), on met à disposition un flux contenant du glycérol produit lors de la préparation d'esters alkyliques d'acides gras supérieurs par transestérification de triglycérides d'acide gras.

4. Procédé selon l'une quelconque des revendications 1 à 3, le flux contenant du glycérol présentant une teneur en eau d'au plus 18% en poids, de préférence d'au plus 15% en poids, en particulier d'au plus 10% en poids.

5. Procédé selon l'une quelconque des revendications 1 à 3, le flux contenant du glycérol présentant une teneur en eau d'au plus 3% en poids.

6. Procédé selon l'une quelconque des revendications précédentes, dans lequel, dans l'étape a), on soumet le flux contenant du glycérol à un traitement par au moins un procédé de traitement qui est choisi parmi un traitement thermique, une adsorption, un échange d'ions, une séparation sur membrane, une cristallisation, une extraction ou une combinaison de deux de ces procédés ou plus.

7. Procédé selon l'une quelconque des revendications précédentes, dans lequel on soumet, dans l'étape a), le flux contenant du glycérol à une distillation pour diminuer la teneur en eau et/ou pour éliminer des composants qui compromettent l'hydrogénation catalytique.

8. Procédé selon l'une quelconque des revendications précédentes, dans lequel, dans l'étape a), on soumet le flux contenant du glycérol à une désulfuration catalytique, le cas échéant en présence d'hydrogène, pour diminuer la teneur en composés soufrés, en particulier en composés soufrés aromatiques.

9. Procédé selon l'une quelconque des revendications précédentes, dans lequel, dans l'étape a), on met en contact le flux contenant du glycérol avec au moins un adsorbant pour éliminer des composants qui compromettent l'hydrogénation catalytique.

10. Procédé selon l'une quelconque des revendications précédentes, dans lequel la préparation du flux contenant du glycérol dans l'étape a) comprend les étapes suivantes :
a1) mise à disposition d'un mélange de départ biogène contenant des graisses et/ou des huiles,
a2) transestérification des triglycérides d'acides gras contenus dans le mélange de départ avec au moins un monoalcool en C₁-C₉ et, le cas échéant, estérification des acides gras libres contenus dans le mélange de départ avec formation d'un mélange d'estérification,
a3) séparation du mélange d'estérification avec obtention d'au moins une fraction enrichie en esters monoalkyliques en C₁-C₉ et d'au moins une fraction enrichie en glycérol libéré lors de la transestérification.
a4) le cas échéant purification de la fraction enrichie en glycérol.

11. Procédé selon l'une quelconque des revendications 1 à 10, le catalyseur utilisé dans l'étape b) comprenant les métaux suivants : Cu, Al, La.

12. Procédé selon l'une quelconque des revendications précédentes 1 à 10, le catalyseur d'hydrogénation utilisé dans l'étape b) contenant au moins 23% en poids, de préférence au moins 35% en poids, de cuivre, sous forme oxyde et/ou élémentaire, par rapport au poids total du catalyseur.

13. Procédé selon l'une quelconque des revendications précédentes, dans lequel on soumet le flux contenant du glycérol dans l'étape b) à une hydrogénation continue dans au moins deux réacteurs d'hydrogénation disposés l'un derrière l'autre.

14. Procédé selon la revendication 13, deux réacteurs à lit fixe disposés l'un derrière l'autre étant utilisés pour l'hydrogénation dans l'étape b).

15. Procédé selon la revendication 14, le premier réacteur d'hydrogénation disposant d'un flux guidé dans un circuit externe, provenant de la zone de réaction.

16. Procédé selon l'une quelconque des revendications 14 ou 15, la transformation dans le deuxième réacteur étant réalisée de manière adiabatique.
